# NEUE EUROPÄISCHE PATENTSCHRIFT

(11) **EP 0 855 002 B2**
(45) Veröffentlichungstag und Bekanntmachung des Hinweises auf die Entscheidung über den Einspruch: **21.12.2011**
(45) Hinweis auf die Patenterteilung: 25.06.2003
(21) Anmeldenummer: 96934667.5
(22) Anmeldetag: 12.10.1996
(51) Int. Cl.: F16M 11/12

(54) **STATIV**
STAND
SUPPORT

(30) Priorität: 12.10.1995 CH 297695
(43) Veröffentlichungstag der Anmeldung: 29.07.1998
(73) Patentinhaber: Leica Microsystems (Schweiz) AG, 9435 Heerbrugg (CH)
(72) Erfinder: METELSKI, Andreas, CH-8590 Romanshorn (CH)
(74) Vertreter: Rosenich, Paul
(86) Internationale Anmeldenummer: PCT/EP1996/004454
(87) Internationale Veröffentlichungsnummer: WO 1997/013997

(56) Entgegenhaltungen:
- EP-A- 0 476 551
- EP-A- 0 628 290
- EP-A- 0 656 194
- DE-A- 4 320 443
- DE-U- 8 400 384
- JP-A- 4 142 507
- US-A- 3 891 301

## Beschreibung

Bei bekannten Stativen mit verstellbarem Gewichtsausgleich sind manchmal Konstruktionen bekannt geworden, die über zwei scheinbar getrennte Teilgewichte verfügen, die voneinander unabhängig verstellbar sind. In der EP-A-628 290 ist beispielsweise ein solcher Aufbau dargestellt. Beide Gewichte sind jedoch um ein gemeinsames Drehlager - dem Hauptdrehlager - bzw. um die Hauptschwenkachsen die nach Erkenntnissen der Erfindung eigentlich nur für das vertikale Schwenken als Lager dienen sollte (als Vertikalschwenkachse des Stativs), zwangsgekoppelt schwenkbar. Das Bewegen eines Gewichtes führt somit automatisch zu einem Bewegen des anderen Gewichtes. Dieses war im Stand der Technik auch beabsichtigt und erwünscht, da man damit versuchte, einen automatischen Gewichtsausgleich über den ganzen Arbeitsbereich des Stativs zu erzielen, sofern einmal eine Gewichtsänderung auf der Seite der Last beim Ausgleichsgewicht berücksichtigt wurde.

Dem dabei angestrebten Gewichtsausgleich liegt die Theorie zugrunde, dass ein optimaler Gewichtausgleich nur dann erzielt werden kann, wenn Lastangriffspunkt, Hauptschwenkpunkt und Schwerpunkt des oder aller Ausgleichsgewichte(s) auf einer Geraden liegen. Diese Theorie ist besser erläutert bzw. ersichtlich in der jüngeren EP-A-656 194. Dort wurde im Unterschied zur Offenbarung der ersterwähnten Lösung mit enormem getriebetechnischen Aufwand versucht, ein einziges Ausgleichsgewicht direkt auf der erwähnten Geraden zu verschieben. Indirekt erfolgte dies jedoch bereits bei der ersterwähten Lösung, indem dort infolge der mechanischen Verbindung der Teilgewichte stets dessen gemeinsamer Schwerpunkt für die Ausgleichs- bzw. Balancierwirkung entlang der fiktiven Geraden verschoben wurde. Diese theoretische Überlegung gilt jedoch in der Praxis nur für ein bestimmtes Gewicht der Last. Unterschiedliche Gewichte verschieben den Lastangriffspunkt neben diese Gerade. Eine Unbalance stellt sich ein.

Auch die Firma Contraves brachte ein Mikroskopstativ auf den Markt mit zwei getrennten Ausgleichsgewichten, wobei eines am ausgleichskraftübertragenden horizontalen Parallellenker in horizontaler Richtung und das andere an eben diesem in vertikaler Richtung verchiebbar ist. Ein solches Stativ ist beispielsweise auch in der EP-B-476 551 beschrieben. Die Technik entwickelte sich - da diese Lösung alter ist - somit anscheinend in richtung Vereinfachung von zwei auf nur ein Ausgleichsgewicht, dafür aber mit deutlich mehr Getriebeaufwand, wie schon erwähnt.

Ein Stativ entsprechend der jüngeren EP-A wurde durch die Anmelderin dieser EP-A (Mitaka) gemeinsam mit der Anmelderin auf den Markt gebracht. Entgegen der Lehre zeigte sich bei diesem Stativ jedoch, dass eine einmal hergestellte Balance nur in einem beschränkten Bereich der Stativsteitung hielt. Wurde das Stativ bzw. die darauf montierte Last an einen anderen Ort geschwenkt, konnte bereits wieder ein Ungleichgewicht fastgestellt werden, was bei Bedienpersonen entweder zu Ermüdungserscheinungen oder zu erhöhtem Justieraufwand während der Arbeit mit dem Stativ führte. Lediglich bei einem ganz bestimmten Gewicht der Last war diese Veränderung gering.

Diesen Nachteil zu beseitigen, ist eine Aufgabe der Erfindung. Es soll ein echt optimal wirkendes System der Balancierung gefunden werden, das die Balance über weite Arbeitsbereiche des Stativs aufrecht erhält, so dass ein Justieren während der Arbeit entfallen kann bzw. nur dann notwendig wird, wenn an der Last selbst das Gewicht geändert wird. Dazu wird auch eine Alternative mit sich ändernden geometrischen Abmessungen der Stativarme vorgeschlagen. Die Bedienperson am Mikroskop soll dabei von Balancierarbeit freigestellt sein.

Diese Aufgabe wird erfindungsgemäß durch die im Kennzeichen des Patentanspruchs 1 angegebenen Merkmale und die in den Kennzeichen der Patentansprüche 18 und 20 angegebenen Verfahrensschritte gelöst.

Die DE-A1-43 20 443 gibt zwar ein semi-automatischen - insofern auch fernbedienbares- Stativ an, bei dem jedoch eine andere Gerwichtsaufteilung von zwei verstellbaren gewichten vorgesehen ist. Desweiteren ist der Aufbau gemäss diesem Dokument auf eine Balancierung in einer bestimmten Lage des Stativs eingeschränkt. Zudem sind zwingend X-Y-Verstellungen der Last (Operationsmikroskop) vorgesehen. Im Zuge der Weiterentwicklung der Erfindung werden auch Verbesserungen gegenüber diesem bekannten Aufbau realisiert.

Die US-A-3 891 301 gibt zwar ein Stativ mit ähnlicher Ausgleichsgewichtsaufteilung an, jedoch ist dieser Aufbau hinsichtlich der nur händischen Ausbalancierbarkeit für die praktische Anwendung umständlich. Der Fachmann sieht andererselts keine Kombinationsmöglichkeit der Lehren dieser beiden zuletzt genannten Dokumente.

Unter vertikaler Schwenkbewegung im Sinne der folgenden Beschreibung ist immer eine Schwenkbewegung eines (vertikalen) Bauteils um eine horizontal liegende Schwenkachse (die Vertikalschwenkachse) des Stativs bzw. Ständers gemeint, die sich zu beiden Seiten einer vertikalen Ebene erstreckt, in der auch diese Vertikalschwenkachse liegt. Unter horizontaler Schwenkbewegung im Sinne der nachfolgenden Beschreibung ist immer eine Schwenkbewegung um eine parallel zur Vertikalschwenkache liegende Schwenkachse (Horizontalschwenkachse) zu verstehen, die gegebenenfalls von dem vertikalen Bauteil getragen wird, wobei sich die Schwenkbewegung zu beiden Seiten einer im wesentlichen horizontalen Ebene abspielt, die senkrecht auf der erwähnten vertikalen Ebene steht, welche die Achse aufnimmt.

Die Ausführungsform zielt auf ein Stativ mit vollautomatischer Justierung der Balance. Die Anwendung des neuen Statives ist nicht eingeschränkt. Insbesondere der optische Bereich Nah- und Fernvergrösserungen fällt darunter. Durch diese Ausbildungsformen bzw. Weiterentwicklungen werden jeweils auch z.T. eigenständige Probleme gelöst, auf die im Folgenden eingegangen wird:

Aus der bereits erwähnten EP-A-656 194 ist andeutungsweise bekannt geworden, dass es Überlegungen gibt, ein Stativ mit einer automatichen Mess- und Verstelleinrichtung auszurüsten (vgl. Spalte 2, Zeile 57, bis Spalte 3. Zeile 5).

Die praktische Ausführung, wie sie in der EP-A-656 194 dargelegt ist (vergleiche Fig.9 bis 13), ist offensichtlich nicht praktikabel, weshalb in dem bestehenden Produkt der Anmelderin dieser Europäischen Patentanmeldung diese automatische Mess-/Einstellvorrichtung nicht eingebaut werden konnte.

Der Gedanke, der offensichtlich im erwähnten Stand der Technik vorlag, war der, eine Unbalance zu erfassen und gemäss dieser das (einzige) Ausgleichsgewicht zu verschieben, um die Balance herzustellen. Die damals vorgeschlagene Vorrichtung verfügte demgegenüber über eine Art Zeiger. Vergleichbar einer Waage wird das Auspandeln (zueinander Bewegen zweier miteinander über eine Kupplung verbindbarer Teile) in die eine oder andere Richtung (Balance zu Gunsten oder zu Ungumsten der Last) angezeigt. Ein photooptisches Element stellt dabei fest, ob der Zeiger in der neutralen, einer linken oder einer rechten Endposition liegt. Die neutrale Position entspricht theoretisch einer Balance, während die linke oder rechte Position einer Unbalance entspricht. Auf Grund dieser information sollte der Stellantrieb für das Ausgleichsgewicht angesteuert werden.

Nachteiligerweise können auf Grund dieser Ja-Nein-Informationen keine spezifischen quantitativen Informationen über die Unbalance gewonnen werden.

Quantitative Informationen jedoch würden as nach Erkenntnis der Anmelderin erlauben, gezielte Verstellungen vorzunehmen. Das ist das Ziel eines weiteren Erfindungsaspektes. Demzufolge sieht die Erfindung neu vor, dass Kräfte oder Momente, die sich aus der Unbalance ergeben, gemessen werden, um eine gezielte Verstellung des oder der Ausgleichsgewichte vornehmen zu können.

Durch diese Massnahme ist ein Schaukeln des Verstellvorganges vermieden, welches gegebenenfalls bei dem vorgeschlagenen system auftreten könnte. Das Ausgleichsgewicht wird erfindungsgemäss auf jene Position verschoben, in der der Messwert der Kräfte oder Momente resultirend aus der Unbalance annähernd Null ist. Dies müssen nicht notwendigerweise absolute Werte sein (absolut Null) es können auch relative Werte sein. Beispielsweise bei einer Biegemessung an einem Lastarm kann im unbelasteten Zustand ein bestimmtes Biegermoment festgestellt werden, das sich durch Erhöhung der Last erhöht. Die Verstellung des Ausgleichsgewichtes wird erfindungsgemäss durch die Grösse des Biegemomentunterschiedes zwischen unbelastetem und belastetem Zustand gesteuert bzw. getriggert. Germäss einer Ausgestaltung der Erfindung ermöglicht ein grosser Unterschied in den Messwerten eine schnelle Verstellung des Ausgleichsgewichtes bzw. der Ausgleichsgewichte; nur geringe Unbalancen hingegen bewirken eine geringfügige Vorschubgesschwindigkeit des Ausgleichsgewichtes.

Die gezielte Steuerung des Ausgleichsgewichtes kann einerseits durch z.B. lichtoptische, balkenkodierte Wegmessgeber oder Distanzmesser am Ausgleichsarm oder aber auch durch eine Drehüberwachung der Antriebsspindel für das Ausgleichsgewicht realisiert sein. Die genaus Kenntnis der Position des Ausgleichsgewichtes vor Beginn einer Messung bei dieser Variante kann durch eine automatische Nulljustierung des Systems (Ausgleichsgewicht fährt automatisch an eine bestimmte Position nach dem Einschalten des Systems) oder auch fliegend bewerkstelligt werden, indem die aktuelle Position das Ausgleichsgewichtes in Bezug auf einen Fixpunkt am Ausgleichsarm ermittelt wird. Bei der Ausbildung der Erfindung mit zwei voneinander unabhängigen Gewichten zum Ausbalancieren der vertikalen und horizontalen Schwenkbewegung geht die Erfindung von einer neuen Erkenntnis aus, nach der vor der Balanceeinstellung für die horizontale Schwenkbewegung eine bestimmte Ausgleichsgewichtseinstellung für die vertikale Schwenkbewegung erforderlich ist. Hierüber gibt es mathematische Zusammenhänge, die je nach Art der Anordnung der beiden Ausgleichsgewichte zueinander in einer Formel oder in einer Tabelle ausgedrückt werden könnten.

Daraus ergibt sich der weiters Erfindungsaspekt, nachdem die automatische Verstellung des einen Ausgleichsgewichtes zu einer ebenfalls automatischen Verstellung des zweiten Ausgleichsgewichtes führt.

Besonders vorteilhaft werden die erfindungsgemäss vorgesehenen Messeinrichtungen als Drehmomentmesseinrichtungen aufgebaut, die im Bereich von beliebigen Achsen des Stativs untergebracht werden könenn, sofern an diesen Achsen zwei Bauteile gelagert sind, die in Abhängigkeit von der Unbalance eine Schwenkbewegung zueinander auszuführen suchen. Denn an diesen Stellen kann die die Schwenkbewegung erzeugende Kraft (das Ungleichgewicht) z.B. als Drehmoment gemessen werden. Erfindungsgemäss wird dabei eine Bremse aktiviert, die die Schwenkbewegung der Teile zueinander blockiert. Die Kräfte, die die Bremse dabei aufnehmen muss, entsprechen den gesuchten Drehmomenten. Im unbalancierten Zustand sind die Torsionskräfte an der Bremse Null. Mit anderen Worten, es findet derart erfindungsgemäss eine Vergleichsmessung der resultierenden Drehmomente aus den Biegemomenten der Last und dem Biegemoment des Ausgleichsgswichtes statt.

Bei einer Variante der Erfindung werden die Kräfte an der Bremse über einen Messarm, der mit einem der beiden zueinander schwenkbaren Teile drehstarr bzw. eingebremst verbunden ist, nach aussen übertragen und von einer starren Abstützung des anderen der beiden Teile abgefangen. Die sich dabei am Messarm ergebende Biegung, die proportional der Torsion ist, wird direkt oder indirekt am Messarm gemessen. Bevorzugt kommen hierfür Dehnmessstreifen-Elemente zur Anwendung. Gemäss einer Weiterbildung der Erfindung kann der Messarm auch ein geringes Spiel gegenüber dem Anschlag aufweisen, so dass geringe Schwenkbewegungen noch ohne Messwerte ablaufen, während andererseits, gegebenenfalls in Abhängigkeit von der Grösse der Messwerte, ein unterchiedlicher Verstellvorgang des oder der Ausgleichsgewichte getriggert werden kann.

Als weitere Ausführungsvarianten sind kombinierte denkbar, bei denen ab einer bestimmten Auslenkung eines Messarmes ein Mikroschalter o.dgl. als Noteschalter betätigt wird, so dass bei übermässiger Unbalance (z.B. ein Operateur stolpert und fängt kurzfristig sein Körpergewicht am Mikroskop ab) das Ausgleichsgswicht sofort in eine neue Augleichsposition verschoben wird. Bei Bedarf kann ein solcher Mikroschalter auch federbelastet und/oder vorgespannt sein.

Es können aber auch Biegungen, die durch die Last am Lastarm auftreten, direkt gemessen werden, indem Dehnmessstreifen-Elemente direkt am Lastarm oder am Ausgleichsarm aufgebracht werden. Balancemessungen mit einer lichtoptischen Libelle einer Wasserwasge sind für Messungen im nicht gebremsten Zustand auch denkbar. Ein Lichtstrahl wird durch eine dunkle Flüssigkeit einer Libelle gedämpft. Lediglich an der Luftblase o.dgl. kann Licht gut durchscheinen. Hinter der Libelle ist ein flächiger Lichtsensor angeordnet, der somit den Stand der Luftblase erfassen und an entsprechende Steuerelemente weiterleiten kann.

Im Rahmen der Erfindung liegen aber auch zusammengesetzte Messmethoden, bei denen z.B. an einer Stelle am Sockel des Stativs der vertikale Gewichtsvektor des Stativs oder eines Teiles davon gemessen wird, während gleichzitig z.B. die Winkelstellung des Lastarmes relativ zur Vertikalen gemessen wird, um daraus auf des Gewicht oder die Gewichtsänderung der Last, die als Komponente den Gewichtsvektor an der Messstelle beeinflusst, rückzurechnen.

Varianten, in denen ausschliesslich die absolute Gewichtsänderung des Gessamtstativs gemessen wird, um die Gewichtsänderung der Last zu ermitteln und in Abhängigkeit davon das oder die Ausgleichsgswichte zu verschieben, sind ebenso durch die Erfindung erfasst. Bei solchen Varianten werden beispielsweise als zweite Messparameter die Relativpositionen der Ausgleichsgewichte zu ihren zugeordneten Schwenkpunkten erfasst, so dass im Falle einer Absolutgewichtsänderung des Stativs, die im Falls des Beispiels nur auf eine Änderung des Gewichtes der Last zurückzuführen ist, die Relativposition des betreffenden Ausgleichsgewichtes positionsgenau verändert wird.

Gemäss einer besonderen Weiterbildung der Erfindung findet die Messung und/oder Verschiebung des Ausgleichsgewichtes zeitverzögert statt. Dies bringt den Vorteil mit sich, dass die Balance relativ unverfälscht hergestellt werden kann. Im anderen Fall ist nämlich davon auszugehen, dass während der Gewichtsänderung der Last (z.B. Auswechseln eines Objektives) eine Bedienperson an der Last (Mikroskop) eine Hand auflegt und diese auch noch kurz nach der Laständerung dort verbleibt, bzw. ein Operateur, nachdem er die elektrischen Bremsen des Stativs gelöst und wieder angezogen hat, mit seinen Händen das Mikroskop noch hält, so dass das Gewicht der Hände das Messergebnis verfälschen könnte.

Alternativ zur eben erwähnten Variante sind jedoch auch Ausführungsformen möglich, bei denen eine permanente Nachjustierung der Ausgleichsgewichte vorgenommen wird, wobei sich daraus ergibt, dass die Präzision des Verschiebevorganges von untergeordneter Bedeutung ist und dass allfällig auftretende extern induzierte, ruckartige Bewegungen des Stativs sofort kompensiert werden; das heisst, dass auch vorübergehende Laständerungen durch Trägheitsmomente, Handanlegung o.dgl., laufend kompensiert werden könnten.

Es versteht sich von selbst, dass im Rahmen der Erfindung auch Varianten liegen, mit unterschiedlichen Verschiebeschwindigkeiten für die Ausgleichsgewichte (z.B. kann jeder Verschiebevorgang nach einer Art Rampe abfahren, mit einer anfänglich grossen und sich stufenweise oder kontinuierlich verringernden Geschwindigkeit), wobei die günstigerweise von den analogen Messwerten der Messeinrichtung gesteuert sein können, was sich als besonderer Vorteil gegenüber dem bekannten Stand der Technik nach der erwähnten EP-A-656 194 ausnimmt, wo praktisch nur Ja-Nein-Entscheidungen gefällt werden können.

Ein weiterer erfinderischer Aspekt der Anmeldung besteht in der Wahl eines neuen vorteilhaften Materials für das Stativ. Der Nachtell bei herkömmlichen Stativen liegt in einer relativ voluminösen Ausgestaltung, die zu einem relativ hohen Gesamtgewicht bei vertretbarer Festigkeit des Stativs und damit zu einer entsprechenden Belastung von Boden oder Decke (je nach Aufstellungsort) führt. Nachteilig ist bei den bekannten Stativen darüber hinaus der Platzbedarf für Last- und Ausgleichsarme, da die geometrischen Abmessungen derselben den Arbeitsspielraum eines Anwenders einschränken. Erfindungsgemäss werden Teile des Stativs zur Reduktion des Gewichtes und Erhöhung seiner Festigkeit aus gesintertem oder geklebtem - vorzugsweise nicht epoxy-gebundenem - Faserverbundwerkstoff z.B. aus Karbon bzw. Kohlenstoffasem, Aramidfasern oder Kevlar (registrierte Marke der Firma Du Pont) aufgebaut; insbesondere betrifft dies den Last- und/oder Ausgleichsarm. Als besonderer Vorteil wurde erkannt, dass die verwendeten Verbundwerkstoffe in Abhängigkeit von ihrer beliebig einstellbaren Strukturierung (Fasernlage) zu den erwähnten Vorteilen führen sowie auch das Schwingungsverhalten der Bauteile positiv beeinflussen, so dass das Stativ direkter positioniert werden kann.

Ein weiterer Aspekt besteht darin, die bisher an Stativen - insbesondere von Operationsmikroskopen - störend vorliegenden Verkabelungen zu entfernen. Bisher war es teilweise bekannt, hohle Arme als Kabelkanäle zu benutzen. Dies hat jedoch den Nachteil, dass bei dem Zusammenbau des Stativs oder in einem Servicefall die Kabel eingezogen bzw. aus den hohlen Armen entfernt werden mussten, was aufwendig ist. Durch diese Massnahme kann die Anzahl der Schnittstellen bzw. Steckverbindungen reduziert werden, so dass die Übetragungsleistung der Kabel optimal ist, obwohl diese gegenüber Zugriff von aussen, wie bei einer Inrohrverlegung gut geschützt sind.

Dazu werden Träger des Stativ zweiteilig und parallellegend ausgebildet, so dass zwischen diesen ein Raum entsteht, in dem die Kabel verlegt werden können, wobei der Raum durch wegnehmbare Abdeckplatten abdeckbar ist.

Eine besondere Ausgestaltung der Abdeckungen für den Kabelkanal sieht eine Schnappbefestigung der Abdeckungen vor, die zwischen den bevorzugt rohrförmig runden Armen mit federnden Resttellen, die mit dem Deckel einstückig ausgebildet, z.B. spritzgussgefertigt sein können Anstelle von Schnappbefestigungen sind auch durchgängige Schraubbefestigungen oder Klebeverbindungen möglich.

Ein weiterer Aspekt liegt in einen anderen Schutz bzw. Kabelkanal für die zur Last - in der Regel zum Mikroskop - führenden Kabel, das für sich unabhängig, jedoch auch in Kombination mit dem eben erwähnten Kabelkanal sinnvoll angewendet werden kann. Es handelt sich dabei um einen flexiblen Schlauch, z.B. einen Wellschlauch, der an seinem Eingang und vorzugsweise auch an seinem Ausgang mit einer drehbaren Kupplung versehen ist, so dass bei einer Drehung des Lastarmes um eine vertikale Achse der Wellschlauch die Möglichkeit hat, die Drehung zu einem gewissen Grad mitzumachen, so dass der Schlauch nicht auf Torston belastet wird. Der Vorteil dieser neuen Kabelführung liegt somit insbesondere in einem Schutz der Kabel vor mechanischen Belastungen und Verletzungen der Kabel durch Einwirkung von aussen. Ausserdem reduziert die Anwendung des Schlauches die Gefahr einer ungewunschten Kontaminierung der Kabel mit Keimen o.dgl.

Andererseits kann der Schlauch einfacher- als bisher die Kabel - desinfiziert werden. Er kann problemlos aus einem Material geschaffen sein, dass an seiner Oberfläche grosszügig mittels flüssiger Desinfektionsmittel behandelt werden kann, was bei bestimmten Isolationsmateriallen für die verwendeten Kabel unter Umständen problematisch ist.

Auf eine detailliertere Beschreibung des verwendeten Schlauches kann verzichtet werden, da ein einsetzbares Produkt per es bekannt und auf dem Markt ist. Als Beispiel wird auf den Wellschlauch NW Duplex bzw. Duplex-SVPA verwiesen, der durch die Rohrfabrik Rüschlikon AG angeboten wird.

Ein weiterer Aspekt der Erfindung ist vor allem bei einem Operationsmikroskop auf die Führung bzw. Halterung desselben im Bereich des Lastengriffes gerichtet. Bei dem bereits erwähnten Stativ der Firma Mitaka und bei allen herkömmlichen Stativen versuchte man im Prinzip die Schwerpunktslage dadurch zu erreichen, dass das eigentliche Mikroskop schlittengeführt links und rechts verstellbar montiert ist, um in die Schwerpunktstage zu kommen, und zwar nicht nur links und rechts, sondern auch nach vom und nach hinten. In allen drei Ebenen gab es hier Verstellschlitten, deren Verstellung durch Bedienpersonal relativ komplex und aufwendig ist.

Gemäss einer weiteren erfinderischen Idee werden auch diese Verstellungen automatisiert, indem allfällige Biegungen, Drehmomente oder andere Messparameter am Mikroskop gemessen und enteprechende Verstellungen von Ausgleichsgewichten vorgenommen werden. Unter Umständen könnten bei den Verstellungen in senkrechter Richtung zur Ebene, in welcher der Lastarm liegt, auch Balancen mit einer Wasserwaage mit optischem Abgriff gemassen werden, wobei die Stellung der Libelle mit optischen Sensoren ausgewertet und daraufhin die entsprechende Verstellung eingeleitet wird.

Im eben erwähnten Bereich können herkömmliche Schlittenführungen und/oder herkömmliche Arme bzw. Parallelogrammführungen vorgesehen sein oder bevorzugt ein neuartiger Kettentrieb, der den Verzicht auf Parallelogrammlenker ermöglicht und trotzdem eine gute Lastaufnahme der Last bei möglichst wenig Raumbedarf und Behinderung von Bedienpersonen sicherstellt. Ein solcher neuer Kettentrieb ist in der CH-Patentanmeldung mit dem amtlichen Aktenzeichen: 03351/95 beschrieben.

Ein weiterer Aspekt der Erfindung bezieht auf die Problematik des Transportes von Stativen mit relativ grossen und ausladenden Armen. Bekannt ist, dass Stative über Ständer und Füsse auf Rädern abgestützt sind. Eine Fixierung gegenüber dem Boden erfolgt herkömmilch durch ein Blockieren der Räder oder ein Absenken von Stellfüsschen o.dgl. Diesbezüglich wird beispielsweise auf das DE-U-84 00 384 verwiesen. Beim Verlagern des Stativs wird dieses herkömmlich auf seinen Rädern gerollt. Beim Durchfahren von Türrahmen o.dgl. kann sich die Baugrösse des Stativs hinderlich auswirken. Zwar konnten schon bisher die Last- bzw. Ausgleichsarme in eine Position geschwenkt werden, die den Raumbedarf des Stativ reduzierte, jedoch ist eine Verbesserung wünschenwert.

Gelöst wird diesses Problem erfindungsgemäss durch die konstruktive Möglichkeit des Absenkens des Ständers des Stativs oder der Arms am Stativ gegenüber dem Ständer und/oder die Absenkbarkeit wenigstens eines der den Fuss stützenden Räder, so dass beim Transport das Stativ eine Schieflage erhält, die seine Transporthöhe und den Schwerpunkt gegebenenfalls weiter reduziert.

Im Zusammenhang mit der für die Arbeit mit dem Stativ erforderlichen Drehbarkeit desselben um eine vertikale Achse hat sich die Erfindung mit einem weiteren grundsätzlich unabhängigen Problem auseinandergesetzt und dieses zufriedenstellend gelöst: Die Baugrösse des Stativs wird auch bestimmt von der Baugrösse seiner Bauteile und von der Anzahl der erforderlichen Einzelbauteile. Für die Drehbarkeit des Stativs um die erwähnte vertikale Achse waren bisher zwei je in einer horizontalen Ebene liegende Lager erforderlich, die die Notwendigkeit einer rohrförmigen Hülse mit sich brachten, die die Lager und darin den Ständer aufnahm. Da die Hülse an sich nichts zur Festigkeit in vertikaler Richtung beitrug, jedoch für die Kippmoment-Kraftübetragung bei unbalanciertem Stativ verantwortlich war, musste sie entsprechend massiv und daher auch schwer ausgebildet sein. Das Reduzieren des Hülsendurchmessers und der Ersatz von Kugellagern durch ein normales Gleitlager brachte zwar eine geringe Durchmessrreduktion des Ständers, erhöhte jedoch die Reibung und reduzierte damit den Bedienkomfort.

Demgegenüber sieht die neue erfinderische Lösung vor, den Ständer mittels eines Kreuzrollenlagers gegenüber dem Fuse abzustützen, das vor altern hinsichtlich der geringen Drehzahlen des Stativs um seine vertikale Achse optimal und verschleissfrei verwendet werden kann. Das Kreuzrollenlager ist aufgrund seiner geringen Bauhöhe gut im Fuss des Stalivs integrierbar und das Stativ somit ungehindert und leichtgängig um seine vertikale Achse drehbar. Der Platzbedarf des Ständers ist gegenüber dem Bekannten weiter reduziert.

Ein weiterer Aspekt der Erfindung betrifft die elektromagnetischen Bremsen, die in den Gelenken des Stativs zur Anwendung gelangen, um die Arme bzw. Armteile relativ zueinander zu fixieren. Eine bekannte Bremse ist beispielhaft in der bereits erwähnten EP-A-656 194 in den Fig.12 und 13 dargestellt. Andrückfedern drücken Bremsscheiben an ein Gegenstück, so dass dieses zwischen zwei Bremsscheiben eingeklemmt ist. An einer Seite einer Bremsscheibe ist ein Elektromagnet montiert, der im erregten zustand die Bremsscheibe anzieht und derart das Gegenstück freigibt. Nach Abschalten des Elektromagnetes wird die Bremsscheibe ungebremst durch die Federkraft gegen das Gegenstück gestossen. Beim Zusammenstoss mit diesem ergibt sich ein relativ lautes Geräusch, des in einem Operationssaal als störend empfunden werden kann.

In einer Weiterbildung der Erfindung ist eine Bremse für die Gelenke des Stativs vorgesehen, die bei guten Bremseigenschaften geräuschämer ist. Dies wird dadurch erreicht, dass der Elektromagnet nicht plötzlich, sondern stufenweise entregt wird, so dass die Bremsscheibe von der Federkraft gebremst gegen das Gegenstück gestossen wird.

Eine verbesserte Variante sieht dabei vor, dass anstelle der Feder zum Andrücken der Bremsscheibe ein Permanentmagnet vorgesehen ist, der im stromlosen Zustand die Bremse eingebremst hält. Der Vorteil dieses Aufbaus gegenüber dem Bekannten ist die noch bessers, geräuschlose Steuerbarkeit das Einbremsvorganges und die Reduktion von bewegten, einem Verschleiss unterliegenden Teilen, nämlich den Federn.

Ein weiterer Erfindungsaspekt betrifft die Vereinfachung der Stativbauteile Während herkömmliche Stative eine Vielzahl von unterschiedlichen Bauteilen aufweisen, denen jeweils eine spezielle Funktion zugstellt ist, wird erfindungsgemäss vorgesehen, wenigstens den Lastarm und den ihn stützenden vertikalen Schwenkarm identisch auszubilden, so dass in der Serienfertigung gespart werden kann. Ein im Schnitt C-förmiger Aufbau dieser Teile bewirkt eine entsprechend grosse freie Arbeitszone für die Anwender. Die neue Geometrie hat sich auch vorteilhaft auf die Balancilermöglichkeit ausgewirkt. In der Figurenbeschreibung wird auf weitere identische Bauteile verwiesen.

Die wichtigsten unterschiedlichen und voneinander unabhängigen, einzeln oder in Kombination anwendbaren, erfinderischen Schritte liegen somit zusammenfassend im
a) Auftrennen der beiden Balancierfunktionen in vertikale und horizontale Balance, die grundsätzlich voneinander unabhängig eingestellt werden können;
b) Trennen der beiden Funktionen in zwei unterschiedliche zueinander parallele Ebenen;
c) Verlagern der Ausgleichsgewichte in einen schwerpunkt-tieferen Bereich am Stativ mittels Parallelogrammlenkern;
d) elektrisches bzw, elektronisches- insbesondere quantitatives - Messen und Einstellen des Ungleichgewichtes an den horizontaten Armen bzw. den damit verbundenen Bauteilen;
e) rechnergestütztes Justieren des vertikalen Ausgleichsgewichtes nach Messung der erforderlichen Ausgleichsgewichtsverschiebung des horizontalen Ausgleichsgewichtes;
f) Verändern der Stativgeometrie zur Schwerpunktsverlagerung bzw, zum Gewichtsausgleich über eine Vertikalschwenkachse und
g) werbesserte Materialwahl.

### Figurenbeschreibung

Die Figuren werden zusammenhängengend beschrieben. Die Figurenbeschreibung und die Bezugszeichenliste bilden eine Einheit, die durch die übrigen Teile der Beschreibung und Ansprüche im Sinne einer vollständigen Offenbarung sich gegenseitig ergänzen. Gleiche Bezugszeichen bedeuten gleiche Bauteile. Gleiche Bezugszeichen mit unterschiedlichen Indizes bedeuten ähnliche, funktionsgleiche Bauteile. Die Figuren sind nur beispielshaft und nicht zwingend proportional richtig dargestellt.
- Fig.1: zeigt ein vereinfachtes Prinzipfunktionsblid eines Stativs mit horizontaler Schwenkbarkeit des Lastarmes;
- Fig.2: ein Prinzipbild eines Stativs mit getrennten Schwenkfunktionen, horizontal, vertikal;
- Fig.3: ein Detail einer Variante mit Drehmomentmessung zwischen Lastarm und Ständer bei einem Aufbau nach Fig.1;
- Fig.4: einen Aufbau mit automatisch verstellbaren Ausgleichsgewichten entsprechend dem Prinzip von Fig.2;

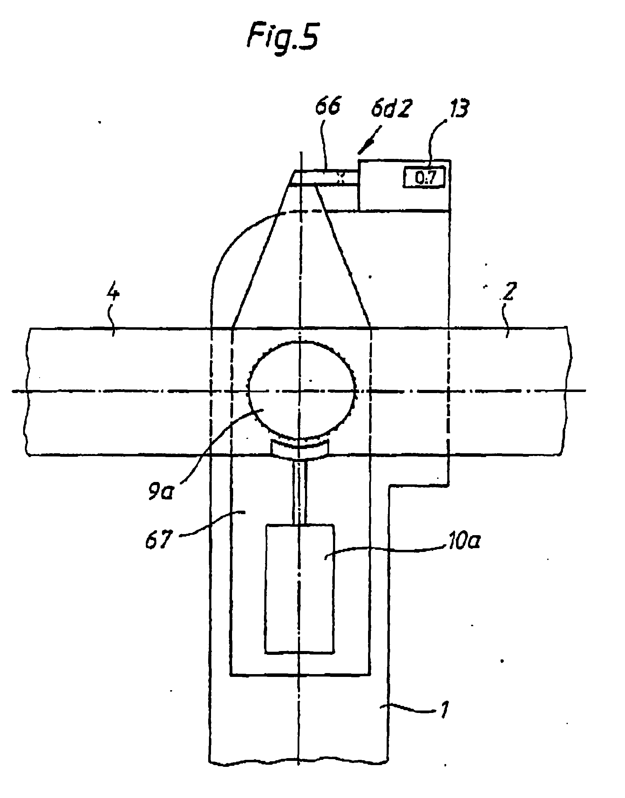

- Fig.5: eine Variante zu Fig.3;
- Fig.6: gestrichen;
- Fig.7: eine Variante zu Fig.6;
- Fig.8: ein Prinzipschaltbild für die erfindungsgemässe Messung und Ansteuerung zweier voneinander getrennter Ausgleichsgewichte;
- Fig.9: den Sockel bzw. Fuss eines erfindungsgemässen Stativs mit neuartiger Drehlagerung;
- Fig.10: den Fuss nach Fig.9 mit seiner Abdeckung:
- Fig.11: eine Schrägansicht einer bevorzugten Ausführungsform der Erfindung:
- Fig.12: dieselbe Ausführungsform wie in Fig.11 aus einem anderen Blickwinkel;
- Fig.13 und 14: Seitenansichten des Aufbaus nach Fig.11 und 12;
- Fig.15: eine Prinzipdarstellung der Funktionen der bevorzugten Ausführungsform nach den Fig.11 bis 14;
- Fig.16: ein Detail mit erfindungsgemässern Kabelkanal:
- Fig.17: eine Ansicht eines einsatzbereiten, erfindungsgemäss verkleideten Stativs;
- Fig.18 und 19: zwei verschiedene Ansichten eines Details der bevorzugten erfindungsgemässen Lösung nach den Fig.11 bis 14 im Bereich einer Bremse und einer Messeinrichtung zur Messung von Drehmomenten, die aus einer Unbalance resultieren und
- Fig.20: gestrichen;

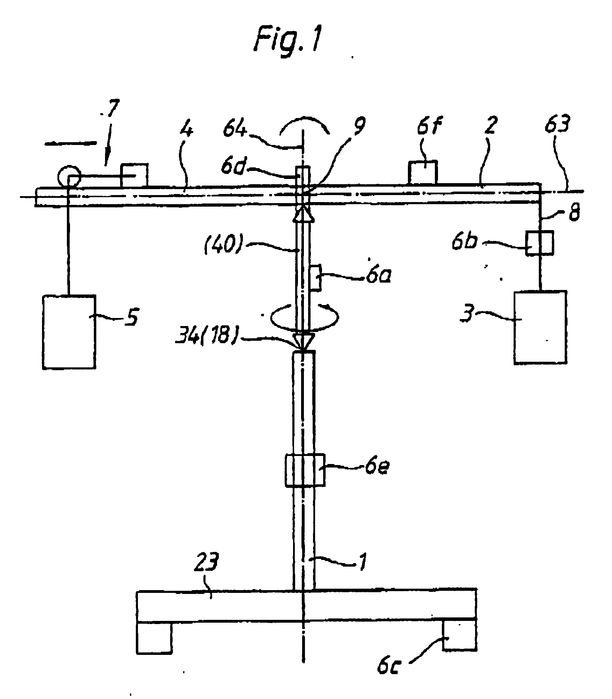

Das waagenähnlich aufgebaute Stativ nach Fig.1 verfügt über eine Schwenkachse 9, über die ein Last- und Ausgleichsarm aus einer horizontalen Ebene 63 geschwenkt werden kann. Ein durch eine Verstelleinrichtung 7 verschiebbares Ausgleichsgewicht 5 versucht, eine Last 3 Ober der Achse 9 auszubalancieren. Erfindungsgemäss wird die Verschiebeeinrichtung 7 durch Messwerte gesteuert, die in Messeinrichtungen 6 a-f gewonnen werden können. Es handelt sich dabei um Messeinrichtungen, die je nach Ort und Art der Montage Biegungen 6a,e,f, Züge oder Drücke 6b,c oder e, oder Drehmomente 6d messen und auf die Verschiebeeinrichtung rückführen.

Sofern die verschiebeeinrichtung entsprechend gesteuert z.B. computergesteuert - ist, können beliebige der erwähnten Messwerte dazu benutzt werden, allfällige Gewichtsänderungen der Last 3 an der Lastaufhängung 6 festzustellen und das Ausgleichsgewicht 5 dementsprechend zu verschieben. Im Falle der Messung von Zügen, Drücken oder Biegung im Lastarm 2 werden zusätzliche informationen benötigt, um die Verschiebeeinrichtung 7 richtig anzusteuern. Diese Informationen können beispielsweise sein; Vor Laständerung Kenntnis der ausbalancierten Position des Ausgleichsgewichtes 5.

Biegemoment-Messungen im Bereich des Ständers 6a,e benötigen insofern keine Zusatzinformatlon, als die Verschiebung des Ausgleichsgewichtes 5 lediglich so zurückgeführt werden muss, dass die Biegemomente gegen Null gehen.

Dasselbe Kriterium trifft auf eine Drehmomentmessung 6d im Bereich des Schwenküberganges zwischen Lastarm 2 und ständer 1 zu. An einem Drehlager 34 sind Last- und Ausgleichsarm 2,4 um eine vertikale Achse drehbar. Eine Vertikalschwenkachse 18 ist im Bereich das Drehlagers 34 engedeutet. Zur Vergrösserung des Aktionsradius des Lastarmes ist es nämlich vorteilhaft, wenn der Ständer zweigeteilt ist und selbst ein Abknicken um eine Vertikalschwenkache 16 ermöglicht. Für verschiedene Varianten und Details der Erfindung ist dies jedoch nicht zwingend erforderlich.

Wenn der obere Teil des Ständers 40 schwenkbar ist, wird er im Folgenden als Schwenkständer bezeichnet. In der Darstellung der Fig. 1 ist es denkbar, die schwenkbarkeit zwischen Ständer 1 und Schwenkständer 40 durch einen Federzug o.dgl. herzustellen, so dass der Schwenkständer 40 durch eine nicht dargestellte Feder im inneren des Ständers 1 in seiner gewünschten Lage im Winkel zur Vertikalen bzw. aus einer vertikalen Ebene 54 herausgehalten wird.

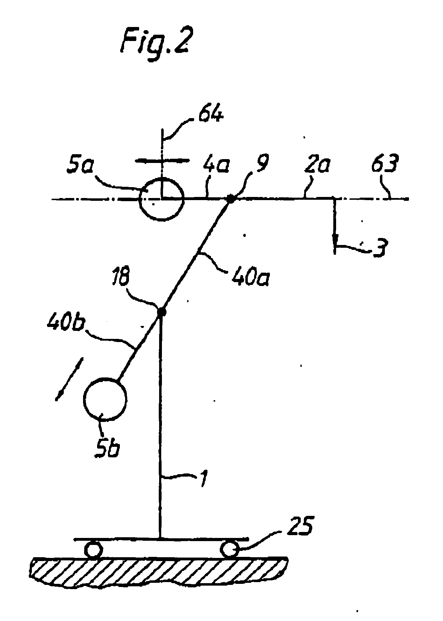

Eine bevorzugte Variante ist demgegenüber in Fig. 2 dargestellt, wo ein Schwenkständer 40 um eine Vertikalschwenkachse 18 frei am Ständer 1 schwerkbar gehalten ist. Ein Teil 40a dient dabei als Lastteil, der die Horizontalschwenkachse 9 und den Last- und Ausgleichsarm 2a, 4a trägt und einen Ausgleichsarm 40b, der ein Ausgleichsgewicht 5b trägt, das den Schwenkständer 40 über der Vertikalschwenkachse 18 in Balance mit dem Ausgleichsgewicht 5a, der Last und dem Gewicht der Arme 2a und 4a hält

Dieses symbolisch dargestellte Stativ stellt die mechanische getrennte Funktion des Horizontal- und Vertikal- Schwenkens um horizontale und vertikale Ebenen 63 und 64 dar.

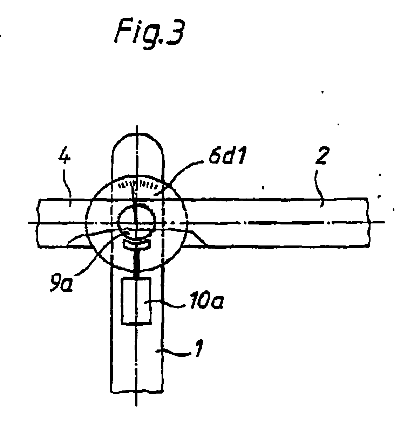

Das in Fig.3 gezeigte Detail zeigt eine Schwenkachse 9A, die grundsätzlich das Freischwenken des Lastarmes 2 gegenüber dem Ständer 1 erlaubt. Am Ständer 1 befindet alch jedoch eine Bremse 10a, die im eingebremsten Zustand die Achse 9a blockiert und ein Verschwenken blockiert. Wird bei einem Obergewicht der Last 3 die Balance in der Achse 9a verlassen, entsteht dort ein Drehmoment, das - wie symbolisch angedeutet - gemessen werden karn. Eine Variante dazu stellt die Messeinrichtung 6d1 jedoch auch eine Winkelmesseinrichtung dar, die für alternative Messmethoden eingesetzt werden kann: Werden beispielsweise die Winkelstellung zwischen dem Lastarm 2 und dem Ständer 1 und der Druck in einer Messeinrichtung 6c (Fig.1) gemessen, so kann aus diesen beiden Werten auf die Grösse der Last bzw, Laständerung geschlossen und dementsprechend eine Verstellung des Ausgleichsgewichtes 5 vorgenommen werden.

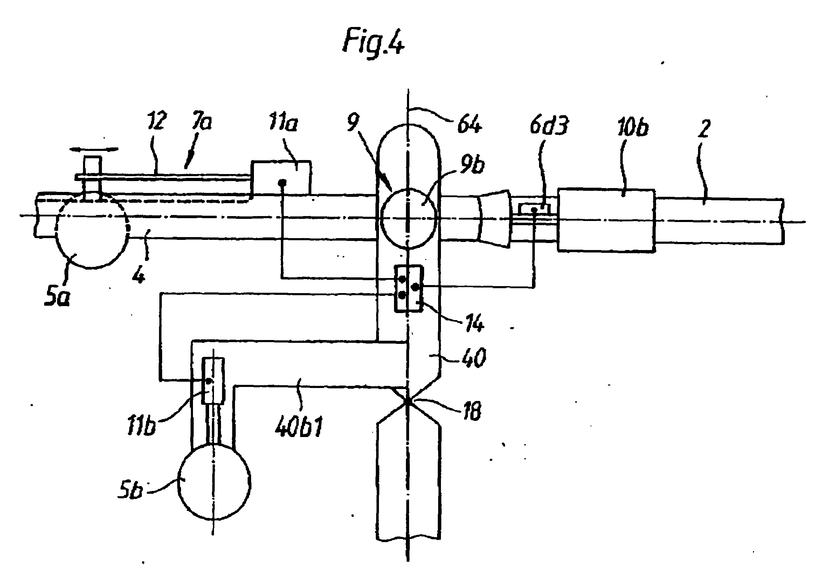

Die Variante nach Fig.4 bezieht sich auf einen prinzipiellen Aufbau gemäss Fig.2 mit Schwankbarkeit sowohl in horizontaler als auch vertikaler Ebene. Der Schwankständer 40 ist bei dieser Ausbildungsform fest mit einer Winkelverlängerung 40b1 verbunden, die ein Ausgleichsgewicht 5b trägt. Ein Schwenken des Schwenkständers 40 aus der Vertikalebene 64 ist möglich wobei das Ausgleichsgewicht 5b die Balance mit den vom Schwenkständer 40 getragenen Bauteilen herstellt. Bei der Variante nach Fig.4 wird mit dar Messeinrichtung 6d3 ein Biegemoment an einem Biegebalken einer Bremseinrichtung 10b gemessen. Ein symbolisch dargestellter Rechner oder Computer 14 wertet das Messergebnis von 6d3 aus und steuert einerseits einen Antrieb 11a der Verschiebeeinrichtung 7a am Ausgleicharm 4 und andererseits den Antrieb 11b am Ausgleichsarm 40b1. In Abhängigkeit von der gewählten Geometrie der Arme gibt es einen Zusammenhang zwischen der erforderlichen Einstellung des Ausgleichsgewichtes 5a und der erforderlichen Stellung des Ausgleichsgewichtes 5b. Erfindungsgemäss wird dieser Zusammenhang im Computer 14 berücksichtigt, so dass eine einzige Messung im Zusammenhang mit der Balance über die horizontale Schwenkebene ausreicht, auch ein erfindungsgemässes automatisches Ausbalancieren für Schwenkbewegungen aus der vertikalen Schwenkebene durchzuführen.

Eine Variante zu Fig.3 ist in Fig.6 dargestellt, wo ein Biegebalken 69 einer Messeinrichtung 6d2 auf Biegung belastet wird, sobald die Bremse 10a, die auf einem Mitnehmerteil 67 montiert ist, diesen auf der Welle 9a einbremst. Symbolisch angedeutet ist eine Anzeige 13, an der die Unbalance abgelesen werden kann. Im Rahmen der Erfindung gegen nämlich auch Varianten, bei denen die Information über die Unbalance einer Bedienperson visuell oder akustisch mitgeteilt werden, um eine manuelle Balancierung zu ermöglichen.

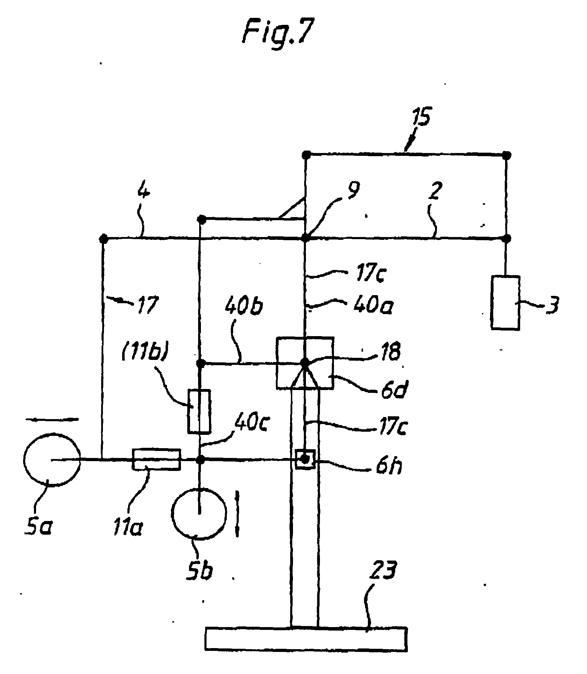

Die Variante nach Fig.7 entspricht dem Prinzip der Fig.2 insofern, als eine vollständige Trennung zwischen Vertikal- und Horizontalschwenkbewegung vorgenommen ist. Der Schwenkständer 40 mit seinem Ausgleicharm 40b trägt das Ausgleichsgewicht 5b, das lediglich um die Vertikalschwenkachse 18 zusammen mit einer entsprechenden Schwenkbewegung des Schwenkständers 40 möglich ist. Demgegenüber ist der Lastarm 2 mit dem Ausgleichsarm 4 bzw, des damit verbundenen Parallelogrammlenkers 15 bzw. 17 so gestaltet, dass ein Schwenken von 15 und 17 um die Horizontalschwenkachse 9 unabhängig vom Schwenkständer 40 möglich ist. Ein Teil des Parallelogramms 17 ist in der Darstellung deckungsgleich mit dem Teil 40a.

Die in dlesem Beispiel vorgesehenen Messmöglichkeiten sind beispielhaft und als Alternative mit Messeinrichtung 6d oder 6h dargestellt.

Ein solcher Aufbau wäre jedoch auch ohne Messeinrichtungen mit rein manualler Verstellung im Rahmen der Erfindung.

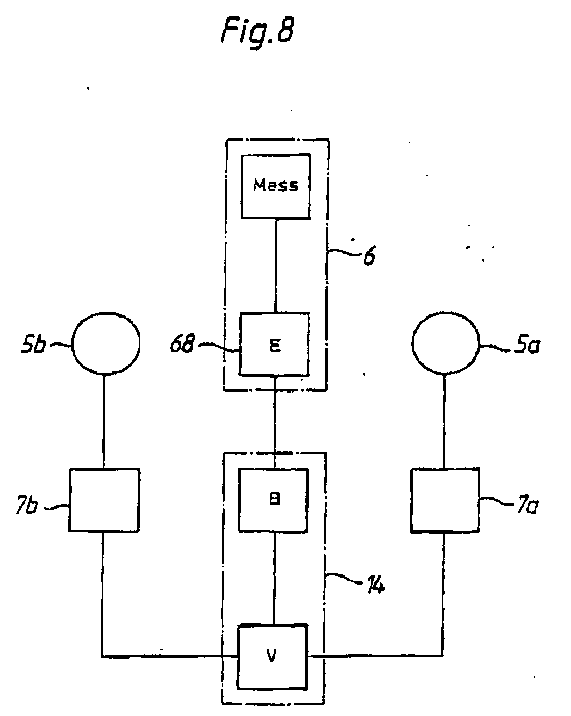

Die neue und erfinderische Verstallmethode ist symbolisch in Fig.8 in einem Blockschaltbild dargestellt. Eine Messeinrichtung 6, die gegebenenfalls eine Positionserfassungseinrichtung 69 umfasst, misst. Unbalance-Parameter. Die Messwerte werden einer Steuerung 14 zugeführt, die einerseits Berechnungen ausführt und andererseits Verstellbefehle herausgibt. Über eine Verstelleinrichitung 7a wird die Position des Ausgleichsgewichtes 6a direkt verstellt. Der zu dieser Verstellung führende Verstellwert wird in der Steuerung 14 mittels Tabellen oder Formeln zu einem Verstellwert für das Ausgleichsgewicht 5b umgerechnet, der durch eine Verstelleinrichtung 7b realisiert wird.

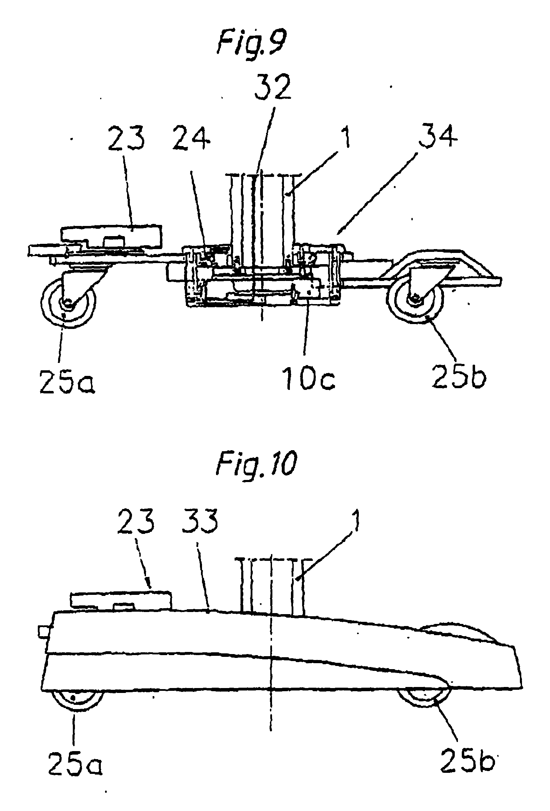

Das Besondere und erfinderisch Neue am Gegenstand der Fig.9 ist u.a. die Verwendung eines Kreuzrollenlagers 24 zur Lagerung das Ständers 1 im Fuss 23. Das Kreuzlager 24 bildet somit das Drehlager 34 für die Drehbarkeit das Stativs um eine vertikale Achse. Räder 25a und 25b sind als drehbare Räder dangestellt. Bevorzugt ist jedoch nur ein Rad oder ein Radpaar um eine vertikale Achse schwenkbar, während ein anderes Rad oder ein anderes Radpaar im Sinne einer bevorzugten Verschieberichtung starr ist

Zur Verhinderung einer ungewünschten Drehbewegung um eine vertikale Achse ist eine Bremseinrichtung 10e im Bereich des Drehlagers 34 vorgesehen, die über eine Versorgungsleitung 32 elektrisch lösbar ist.

Fig.10 gibt ein Gehäuse 33 für den Fuss 23 wieder.

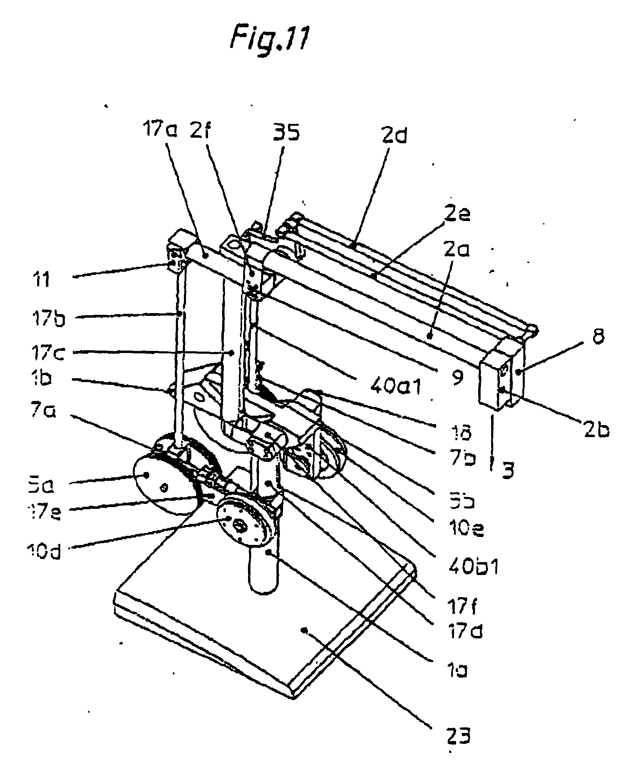

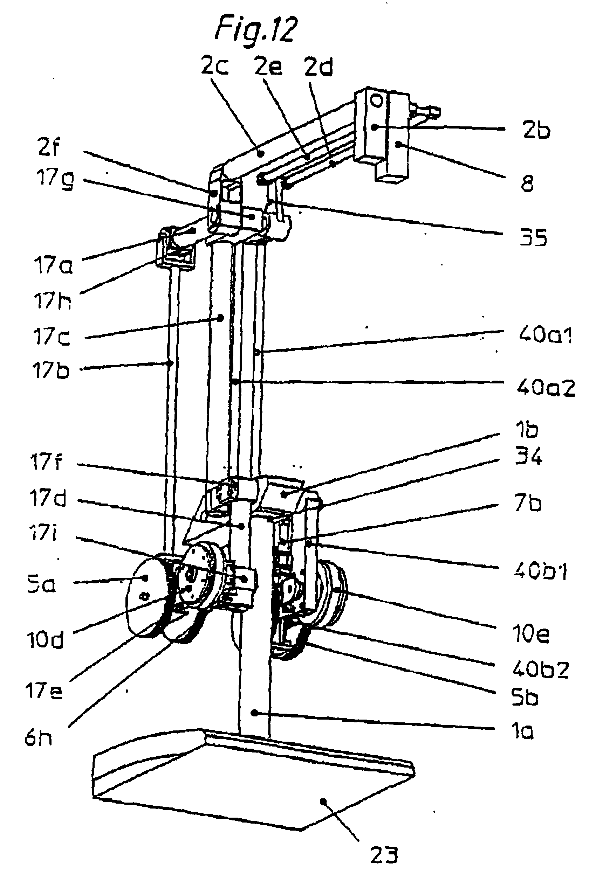

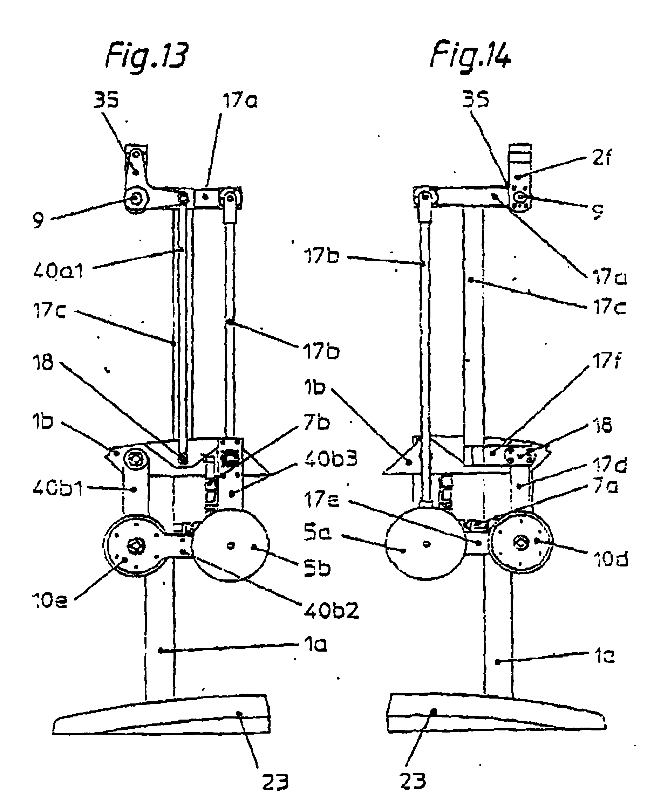

Fig.11 bis 14 zeigen eine bevorzugte Ausfühungsform der Erfindung mit einer Vielzahl identischer Bauteile, was einer rationellen Fertigung entgegenkommt. Eine Parallelogrammführung 17 mit einzeln bezeichneten Bauteilen 17a-f trägt das Ausgleichsgewicht 5a und eine Messeinrichtung 6h. Ein Schwenkständer 40 mit Parallelogrammführung 40b trägt das Ausgleichgewicht 5b. Ein Lagerbook 35 und damit verbundene Arme 2d und e sowie 40a1 und 40a2 sichern bei allen Schwenkbewegungen die senkrechte Lage der Lastaufhängung 8.

Wenigstens einige der folgenden Bauteile sind zur produktionteschnischen Vereinfachung identisch, 17a entspricht 17d; 17g entspricht 2b; 17c entspricht 2c; 17f entspricht 2f; 17h entspricht 171: 2e entspricht 2d; 5a entspricht 5b.

Ein Schwenkkopf 1b, der als Haupttragteil auf einem Drehlager 34 am Ständer 1a sitzt, hält mittels nicht sichtbarer Achse die beiden Schwenkständerarme 40a1 und 40a2 in Position. 40a1 und 40a2 könnten auch als ein einziges Stück ausgebildet sein, ebeneo wie die beiden Armteile 2d und 2e. Erfindungsgemäss sind diese jedoch zweigeteilt, um einerseits die Bautiefe zu reduzieren und andererseits den erfindungsgemässen Kabelkanal zu schaffen.

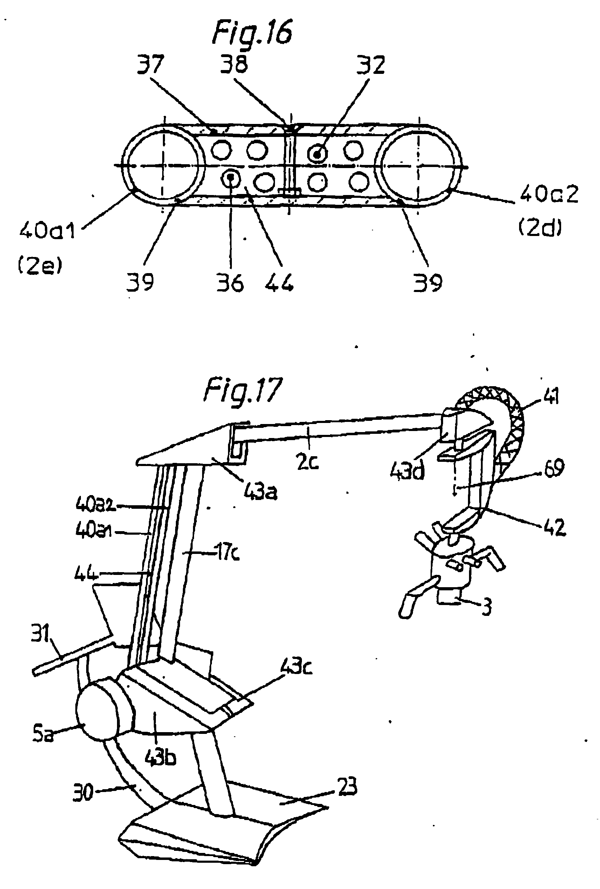

Dieser Kabelkanal ist aus Fig.16 im Schnitt ersichtlich und weist im wesentlichen eine Abdeckung 38 und einen Deckel 37 auf, die den Raum zwischen den beiden rohrförmigen Armen zu einem Kanal abdecken.

Zur Abdeckung 38 und zum Deckel 37 liegen im Rahmen der Erfindung verschiedene Varianten, unter anderem mit Schnappeinrichtungen zum Aufschnappen auf die beilden Rohre.

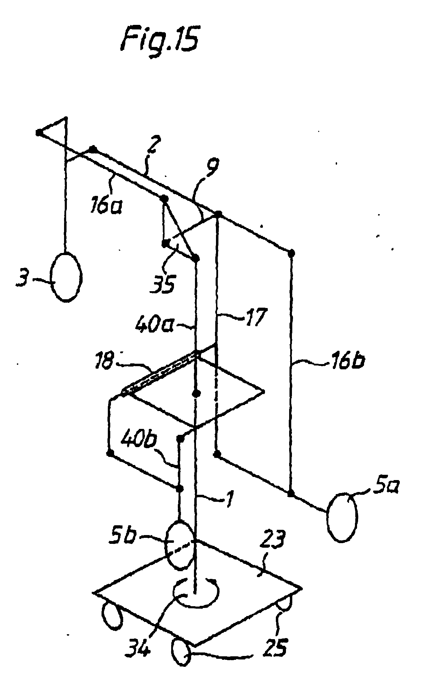

Fig.15 zeigt in symbolischer Darstellung eines der Prinzipien der Erfindung, das auch in den Gegenständen 11 bis 14 realisiert ist

Das Stativ gemäss Fig. 17 haut im Prinzip auf den Fig. 11 bis 14 auf. Erfindungsgemäss sind hier Jedoch die Kniestellen-bzw, Gelenke- des Stalivs durch Abdeckkappen insbesondere aus Polyurethan-Integralschaum verdeckt. Als weitere Neuerung ist ein Schlauchkanal 41- vorzugsweise ein Wellschlauch - dargestellt, der in Verlängerung des Kabelkanals zwischen den Armen 2 bzw, 40a Kabel, die zur Last (Mikroskop) 3 geführt werden müssen, schützt, Bevorzugt ragt der Schlauchkanal 41 von der Ebene, in der die Arme 2 liegen, senkrecht sb. Dies ermöglicht das freie Drehen der Last um eine vertikale Drehachse 69. Die Last kann somit bequem in alle gewünschten Richtungen gedreht werden, ohne dass die Kabel oder der Schlauchkanal 41 im Weg sind. Durch den Schlauchkanal 41 kann erfindungsgemäss bevorzugt auch Luft abgesaugt wenden, was zu Kühlzwecken oder Evakuierungen unter einem Drape genutzt werden kann. Gegebenenfalls können solche Absaugvorgänge durch den Kabalkanal zwischen den Armen oder durch die Arme selbst fortgesetzt sein.

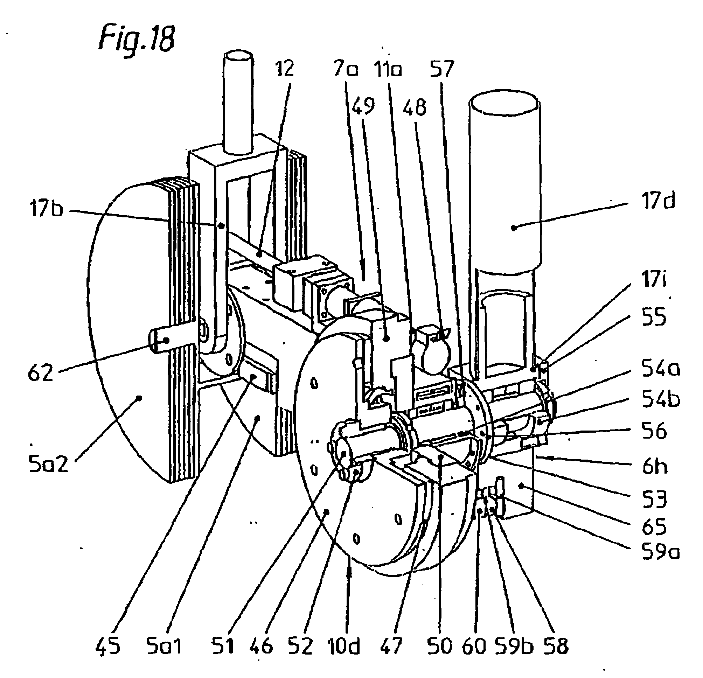

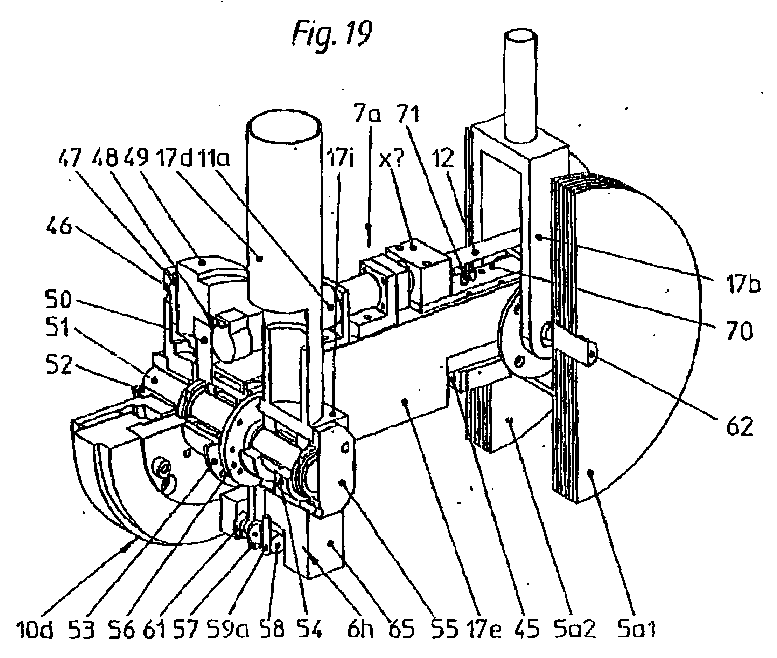

Die erfindungsgemäss Brems- und Messeinrichtung entsprechend der Detailzeichnung in Fig.18 und 19 bzw. Verstellvorrichtung 7a für das Gewicht 5a funktioniert wie folgt: Im nicht erregten Zustand des Elektromagneten 50 wird die Bremscheibe 48 durch den Permanentmagnet 49 über Bremsbacken 47 angezogen und eingebremst. Die Bremsscheibe 46 ist über eine Spannhülse 52 starr mit einer Welle 51 verbunden. Permanentmagnet und Elektromagnet sind über eine Lagerhülse 53, die am Arm 17e angeschraubt ist, starr mit diesem verbunden. Die Welle 51 ist somit in diesem Zustand starr mit dem Arm 17e verbunden. Ein Schwenken des Armes 17d ist über die Lager 54b grundsätzlich möglich. Zwischen dem Teil 171 und der Lagerhülse 53 ist jedoch eine Drehgsberscheibe 56 starr mit der Welle 51 verbunden. Sie trägt starr einen Drehmitnehmer 57, der nach unten regt und mittels Befestigungsmutter 61 einen Biegebalken 58 festhält. Der Biegebalken 58 ist zwischen zwei spielfrei gehaltenen Anschlägen 58 gehalten. Die Anschläge 59 befinden sich starr in einem Messkörper 65 der Messeinrichtung 6h. Leizterer ist zur Verdeutlichung geschnitten dargstellt. Der Messkörper 65 ist starr mit dem Teil 17i und damit mit dem Teil 17d verbunden. Ein Schwenken desselben über seine Lager 54 ist somit angehalten.

Eine Schwenktendenz führt jedoch zu einer Biegebelastung des Biegebalkens 58. Auf diesem sind Dehnmessstreifen 60 montiert, die als Messergebnis elektrische Spannungsimpulse einer entsprechenden Steuerung moderieren.

Selbstverständlich können an Stelle von Dehnmessstreifen und Biegebalken auch andere Messelemente wie beispielsweise Piezoelemente o.dgl. in an sich bekannter Form die beispielhaft dargestellte Variante ersetzen.

Eine bevorzugte Mess- und Steuerroutine ermöglicht erfindungsgemäss das unterschiedlich schnelle Ansteuern der Ausgleichsgewichts. So kann beispielweise bei Spannungswerten zwischen plus/minus 10 Volt der Befehl für eine Schnellverstellung ausgelöst werden, wobei zwischen plus/minus 5 Volt eine normale Verstellgeschwindigkeit induziert wird und im Bereich zwischen plus/minus 2 Volt nach einer Spannungsrampe ein verlangsamtes Fahren zum gewünschten Einstellwert des Ausgleichsgewichtes vorgegeben ist. Ebenso sind im Rahmen der Computersteurung 14 Varianten denkbar, mit Wartezeiten zwischen aktuellen Messergebnissen und aktuallen Verschiebevorgängen. Gemessen wird in der Regel mit angezogenen Bremsen. Gelöste Bremsen ermöglichen einer Bedienperson, die Stellung des Stativs zu verändern.

Bei einer besonderen Ausführungsform der Erfindung sind - abgesehen von Kraftmessungen - auch Absolut-Positionsmessungen der oder wenigstens eines Ausgleichsgewichtes 5a relativ zu seinem Trägerarm 17e vorgesehen. Ein optischer oder magnetischer Balkencode 70 wird durch ein magnetisches oder optisches sensorelement 71 abgetastet. Das Sensorelement ist direkt oder indirekt mit dem Ausgleichsgewicht 5a verbunden, so dass dessen Position am Arm 17e feststellbar ist Absolutpositionsmessungen haben den Vorteil, gemäss einer weiteren Ausbildung der Computersteuerung 14 in Abhängigkeit von Laständerungen mit dem Ausgleichsgewicht 5 gezielt auf eine bestimmte Ausgleichspositionen fahren zu können. Grundeinstellungen bzw. Eichungen sind demzufolge auch möglich. So kann der Steuerung beispielsweise bereits beim Montieren eines Zusatzteiles an der Last (Lasterhöhung) das ungefähre Gewicht dieses Bauteils angeben werden, worauf die Steuerung noch ohne Durchführen einer Messung bei der Messeinrichtung 6a eine Positionierung (Neupositionierung) des Ausgleichsgewichtes vornehmen kann. Eine besonders einfache Variante ist auf jenen Faktor gestützt, der in Abhängigkeit von der Geometrie des Stativs ermittelt wird. Beim Verschieben eines der Ausgleichsgewichte entsprechend einem Messergebnis in die eine oder andere Richtung wird automatisch das andere Ausgleichsgewicht um eine faktoriell verkürzte oder verlängerte Wegstrecke verschoben. Dies kann durch entsprechende Drehzahlregelung der Stellantriebe oder unterschiedliche Gewindesteigungen von Spindein 12 usw. erzielt werden.

### Bezugszeichenliste

- **1a,c:**: Ständer, vorzugsweise am Boden rollbar; ist nur symbolisch mit geradem Stab dargestellt; könnte auch C-förmig, kastenförmig oder vergleichbar aufgebaut sein; muss nicht zwingend für eine Bodenmontage bzw. Aufstellung dienen sondern könnte auch umgekehrt sein und an einer Decke, sonstigen Flächen oder Einrichtungsgegenständen - gegebenenfalls verfahrbar - montiert sein.
- **1b:**: Ständerkopf, ist ein Bauteil, der den Ständer nach oben hin zur Aufnahme der schwenkbaren Teile des Stativs abschliesst und insbesondere selbst drehbar am Ständer 1 sitzt.
- **2,a:**: Lastarm, eventuell aus mehreren Stäben aufgebaut; z.B. eine oder mehrere Parallelogrammführungen.
- **3:**: Last. z.B. Mikroskop, könnte aber auch ein beliebiger Bauteil sein, der an einem Stativ zu halten ist, z.B. Roboterarm, Fernrohr o.dgl.
- **4,a:**: Ausgleichsarm, eventuell aus mehreren Stäben aufgebaut; z.B. eine oder mehrere Parallelogrammführungen.
- **5a-d:**: Ausgleichsgewicht verschiebbar; kann einstückig oder insbesondere getellt sein. Einer von verschiedenen Aspekten der Erfindung ist, dass zwei getrennte Ausgleichsgewichte für zwei von einander bewegungsgetrennte Ausgleichsfunktionen - nämlich um eine vertikale und um eine horizontale Ebene (63) - pendeln.
- **6a-h:**: Messeinrichtung für Kräfte, Kräfteänderungen bzw. Gewichte oder Gewichtsänderungen am Lastarm, insbesondere zur Druck-, Zug-, Biege- oder Drehmomentmessung an Stellen des Stativs, an dem solche Messwerte direkt oder indirekt abgenommen werden können.
- **7a-c:**: Verschiebeeinrichtung für ein Ausgleichsgewicht: umfasst z.B. Gleit- oder Rollanführungen, Antriebsriemen, -spindeln o.dgl. und einen Antrieb 11 sowie geeignete Lagerungen und Verbindungen.
- **8:**: Lastaufhängung; umfasst Vorrichtungen zur Aufnahme eines Mikroskops oder sonstiger Lasten; insbesondere umfasst die Lastaufhängung gemäss einer Weiterbildung der Erfindung auch ein eigenes - dem Balanciersystem des Stativs selbst entsprechendes - Balanciersysterm mit Last- und Ausgleichsarmen sowie Messeinrichtungen und Ausgleichsgewichten.
- **9:**: Schwenkachse (Horizontalschwenkachse) für den Lastarm 2 und/oder Ausgleichsarm 4, an der diese aus einer horizontalen Ebene 63 schwenken können,
- **10a-e:**: Bremseinrichtung zur Abbremsung bzw. gegenseitigen Fixierung von zueinander beweglichen Bauteilen,
- **11a-c:**: Antrieb, vorzugsweise elektrischer, zur Bewegung eines Ausgleichsgewichtes.
- **11 d,e:**: Antriebe zum Verändern von einer stativarmgeometrie, insbesondere Verlängern oder Verkürzen der Arme 17d und 40a3 bzw. 40b3; die erwähnten Arme werden gleichförmig verlängert oder verKürzt; die dafür verwendeten Bauteile sind nur symbolisch dargostellt; einem Fachman bieten sich im Detail dafür verschiedenste Lösungen an wie Paratteispindein bzw. -führungen, Teleskopverbindungen usw.
- **12:**: Antriebsspindel, vorzugsweise selbsthemmende, zum Antrieb eines Ausgleichsgewichtes,
- **13:**: Display; kann herkömmlicher Zeiger oder elektrisches Display sein z.B. CRT, LCD, LED usw, zum fakultativen Anzeigen von Mess- oder Verstellwerten.
- **14:**: Messwertwandler, Steuerung, Logik bzw. Computer usw. erkennt, errechnet und verwendet u.U. das Messergebnis der Messeinrichtung 6 eventuell tabellengestützt - zum positionsbestimmten und/oder geregelten (try and error) Ansteuern des Antriebes 11 zur Positionierung eines Ausgleichgewichtes.
- **15:**: Parallelogrammführung für den Lastarm und entspricht dem Lastarm 2 in einer bevorzugten Ausführungsform; ermöglicht auch das Senkrechthalten des Lasthalteteils 8 seibst bei einer Schwenkbewegung über die Vertikalschwenkachse 18 oder Horizontalschwenkachse 9, wie dies an sich schon bei Tischlampenständem und bei KFZ-Parallellenkem bekannt ist.
- **16:**: Zugarm horizontal (a) vertikal (b).
- **17:**: Parallelogrammführung für den Ausgleichsarm 4 bzw. zur Verlagerung des Ausgleichsarmes und des Ausgleichsgewichtes nach unten, um den Schwerpunkt tiefer zu legen; entspricht dem Ausgleichsarm 4 in einer beavorzugten Ausführungsform.
- **18:**: Drehpunkt bzw. Schnitt durch Drehachse bzw. Schwenkachse (Vertikalschwenkachse), um den das Stativ aus einer vertikalen Ebene schwenken kann.
- **19:**: Winkel- Neigungssensor.
- **19b,c:**: Neigungsesensoren (Wasserwaagen), die im ungebremsten Zustand des Stativs gemäss Fig. 20 in der Lage sind, eine Balance über dem Vortikalschwenklager 18 fastzustellen; eine Balance in der dargestellten Lage - bei vertikalem Arm 40 gemessen am Sensor 19b - bedeutet gute Justierung des Ausgleichsgewichtes 5d. Im Bedarfsfall wird mittels Verstelleinrichtung 7c das Ausgleichsgewicht nach Änderung der Last 3 neu justiert. Ein Stellantrieb 11c wirkt auf das Gewicht 5d beispielsweise mittels Gewindestange. Die Justierung für die Schwenkbewegung aus der Horizontalebene erfolgt durch eine erfindungsmässe Längenverstellung der Arme 17d und 40a3 bzw, 40b3, so dass der Schwerpunkt des Ausgleichsgewichtes 5d bzw, des mit ihm verbundenen Gestanges usw. gegenüber dem Vertikalschwenklager 18 tiefer oder höher gelegt bzw. von diesem Lager 18 entfernt oder angenähert wird. Durch diese neue Massnahme ist es möglich, sich ergebendes Ungleichgewicht bei Schräglage der Arme 40a,b bzw. 17d auszubalancieren. Dieses kann permanent durch kontinuierliche Messung mittels Sensor 19c erfolgen oder auch - wie bei anderen erfinderischen Lösungen - zwangsgekoppelt sein mit der Einstellung des Ausgleichsgewichtes 5d. Der Unterschied dieser Variante liegt somit darin, dass kein eigenes Gewicht für die Vertikalschwenkbalancierung vorgesehen ist, sondern das Horizontalausgleichsgewicht 5d auch für diesen Ausgleich herangezogen wird. Nachteilig ist dabei gegenüber den Varianten mit völliger Trennung zwischen den beiden Ausgleichsgewichten, dass eine einmal eingenommene Ausgleichsstellung nur für eine Last ideal funktioniert: daher wäre bei einem solchen Aufbau eine laufende Justierung bevorzugt.
- **20:**: Linie
- **21:**: Führung
- **22:**: Einstellvorrichtung, z.B. Befehlsknopf und Steuerung.
- **23:**: Fuss des Stativs, dient zur Abstützung auf dem Boden, ist aber auch umgekehrt als Halteteil an einer Decke o.dgl. denkbar mit modifizierten Halteelementen (keine Rollen).
- **24:**: Kreuzrollenlager als Ersatz von zwei Kugel- oder Wälzlagern.
- **25:**: Räder für Fuss, können starr (nur eine bevorzugte Transportrichtung) oder drehbar befestigt sein; sind bevorzugt fixierbar oder durch parallele Aufstellfüsschen vom Boden abhebbar oder gegenüber dem Fuss 23 bzw. In diesen einziehbar, um ein Absetzen des Fusses am Boden zu ermöglichen.
- **26:**: Stellmechanismus, z.B. Stellschraube
- **27a,b,c:**: Pfeile
- **28:**: Hülse; nimmt den Ständer formschlüssig auf und übernimmt gegebenenfalls eine Drehlagerfunktion.
- **29:**: Arretierknopf zum Arretieren der Teleskopverschiebbarkeit des Ständers 1 in der Hülse 28.
- **30:**: Transportgriff zum Schieben oder Ziehen des Stativs; durch eine spezielle Griffstange 31 gibt er bevorzugt eine besondere Transportrichtung (Pfeil 27a) vor.
- **31:**: Griffstange
- **32:**: elektrische oder optische Versorgungsleitung o.dgl. für Funktionen des Stativs, z.B. Bremsen oder der Last (Mikroskop).
- **33:**: Gehäuse des Fusses 23; zur Tieferlegung des Gesamtschwerpunkts des Stativs aus Gussmaterial o.dgl. und/oder aus Kunststoff überzogen bzw. ausgebildet.
- **34:**: Drahlager
- **35:**: Lagerbock
- **36:**: Abdeckung für Kabelkanal zwischen parallelen Armen des Stativs.
- **37:**: Deckel für Kabelkanal.
- **38:**: Befestigungsschraube zum Verbinden von Abdeckung 36 und Deckel 37; die Abdeckung kann gegebenenfalls mit den Armen verklebt sein oder auch nur mittels Schrauben 38 gehalten werden.
- **39:**: Klebestellen
- **40a-c:**: Schwwenkständer
- **40a:**: Schwenkständer; es ist der im Ruhezustand senkrechte Bauteil, der das Horizontalschwenklager 9 trägt bzw. dieses in die Höhe hält; seine Funktion ist es, beim Schwenken aus einer Vertikalebene 64 das Schwenklager 9 und damit den Lastarm 2 seltlich zu verschieben, so dass die Last 3 von der Vertikalebene 64 und auf sie zu bewegt werden kann; er verfügt über eine vertikale Verlängerung unterhalb des Vertikalschwenklagers 18, die als Ausgleichsarm dient und das Ausgleichsgewicht 5b aufnimmt.
- **40b:**: Schwenkständerausgleichsarm; der Schwenkständer 40a und der Schwenkständerausgleichsarm 40b entsprechen von ihrer Funktion her einem Last- und Ausgleichsarm, vergleichbar mit den Armen 2 und 4; dies ist auch eine Besonderheit an der Erfindung, dass die Ausgleichsfunktionen derart vollständig getrennt und über den Schwenkständer 40 mit dem Horizontallager 9 und das Vertikallager 18 wieder integriert sind.
- **41:**: Schlauchkanal - insbesondere Wellschlauch - dient der Aufnahme und dem Schutz von elektrischen oder optischen Versorgungskabeln für die Last 3 und stellt eine Fortsetzung der Kabelkanäle 44 zwischen den Stativarmen dar, er ist insbesondere längsachsen-zweiteilig, so dass er zur entnahme von Kabeln über seine Länge geöffnet werden kann.
- **42:**: Mikrokopaufhängung, könnte eine herkömmliche sein, wie im erwähnten Stand der Technik angegeben, wird jedoch bevorzugt nach einem neuen erfinderischen Prinzip gebaut, das Gegenstand einer am gleichen Tag eingerelchten CH-Patentanmeldung mit dem amtlichen Aktenzeichen: 03351/95 ist, auf die zu Kombinationszwecken ausdrücklich Bezug genommen wird.
- **43a-d:**: Abdeckkappe, aus vorzugsweise allseitig geschlossenem Integralschaum, ist zu Servicezwecken leicht abnehmbar an den Gelenke aufweisenden Stellen des Stativs angebracht und verhindert im Falle von Zusammenstössen Vertetzungen oder Beschädigungen; als weiterer Vorteil ist das geringe Gewicht und die beliebige Formbarkeit herauszuheben, die dem Stativ mit geringen Mitteln auch ein gefälliges Aussehen verleiht
- **44:**: Kabelkanal
- **45:**: Schlittenführung für Ausgleichsgewicht 5 am Ausgleichsarm; in den Figuren ist nur eine Schlittenführung für das Ausgleichsgewicht 5a dargestellt; für das Ausgleichsgewicht 5b ist bevorzugt eine identische Schlittenführung vorgesehen, so dass diese nicht näher angegeben werden muss.
- **46:**: Bremsscheibe einer Bremse 10.
- **47:**: Bremsbacke; ist vorzugsweise ein Polschuh eines Magneten 49, könnte jedoch auch aus einem anderen- nichtmagnetischen - Material aufgebaut sein, um eine zu Metall unterschiedliche Bremswirkung zu erzielen; gegebenenfalls handelt es sich dabei auch nur um einen dünnen Überzug eines Polschuhs eines Magneten 49.
- **48:**: elektrische Steueranschlüsse für den Verstellantrieb 11 der Verstelleinrichtung 7 für die Ausgleichsgewichts 5 und/oder für einen Elektromagnet 50 der Bremse 10.
- **49:**: Permanentmagnet
- **50:**: Elektromagnet, er ist so gepolt, dass er im erregten Zustand die Magnetkraft des Permanentmagneten 49 aufhebt.
- **51:**: Welle in der Bremse 10, dient der Drehmomentübertragung bei eingebremster Bremse 10.
- **52:**: Spannhülse: dient der Befestigung der Bremsscheibe 46 auf der Welle 51.
- **53:**: Lagerhülse; dient der Halterung von Kugellagern 58 über der Welle 51 im Bauteil 17e der Parallelogrammführung 17.
- **54:**: Lager; z.B. Kugel-, Rollen- oder Nadellager, dienen der reibungsminimierten Lagerung der zueinander beweglichen Bautelle bzw. Arme des Stativs; als erfindungsgemässer Effekt ergibt sich daraus eine besonders leichte Handhabung das Stativs bei geöffneten Bremsen, so dass eine Bedienperson - gute Balancierung vorausgesetzt - so wenig wie möglich ermüdet, widerstandsfrei und hochpräzise die Last- insbesondere ein Operationsmikroskop - positionieren kann.
- **55:**: Spanndeckel; dient zum Abdecken des Wellenstummels der Welle 61 und gegebenenfalls zum Spannen der Lager 54b.
- **56:**: Drehgeberscheibe; sie ist mit der Welle 51 starr verbunden und überträgt derart Drehmomente mittels Drehmitnehmer 57 relativ zum auf der Welle 51 frei drehenden Arm 17d der Parallelogrammführung 17.
- **57:**: Drehmitnehmer
- **58:**: Biegebalken; er ist mit dem Drehmitnehmer mittels Mutter 61 starr verbunden und gegenüber dem Arm 17d bzw. Teil 17i an je einem Anschlag 59a,b angeschlagen; die Anschläge 58 sind mit einem Messkörper 65 verbunden, könnten aber auch einstückig mit diesem ausgebildet sein; sie sind bevorzugt rund und liegen spielfrei am vorzugsweise ebenso runden Biegebalken 58 an, so dass Bewegungen zwischen beiden möglichst friktionsfrei erfolgen können.
- **59a,b:**: Anschlag
- **60:**: dehnmessstreifen; er ist am Biegebalken in an sich bekannter Art aufgebracht und erlaubt eine Messung der Biegung desseiben unter der Kraft, die auf ihn über den Drehmitnehmer 57 übertragen wird; das Messergebnis ist proportional zum Drehmoment, das von der Bremse 10d und der Welle 51 gegenüber dem Arm 17d übertragen wird.
- **61:**: Mutter
- **62:**: Aufnahmezapfen
- **63:**: horizontale Ebene
- **64:**: vertikale Ebene .
- **65:**: Messkörper
- **66:**: Biegebalken
- **67:**: Mitnahmetall
- **68:**: Positionserfassung
- **69:**: Vertikale Drehachse
- **70:**: Balkencode
- **71:**: Sensorelement

## Patentansprüche

1. Stativ zum Tragen einer Last (3), insbesondere eines Operationsmikroskops, mit einem Ständer (1) mit wenigstens einem Lastarm (2) für die Last (3) und wenigstens einem Ausgleichsarm (4) für ein Ausgleichsgewicht (5) und mit mindestens einer Drehachee (18), um die der lastarm (2) und der Ausgleichsarm (4) schwenkbar gelagert ist, wobei eine Bremse (10) zur Arretierung der Bewegung des Lastarms (2) und des Ausgleichsarms um die Drehachse (18) vorgesehen und das Ausgleichsgewicht (5) entlang des Ausgleichsarms (4) verschieblich ausgebildet und in zwei Teilgewichte (5a, 5b) unterteilt ist und zum automatischen Ausbalancieren von Gewichtsänderungen am Lastarm (2) oder des Lastarmse (2) gegenüber dem Ständer (1) eine Messeinrichtung (6) zur Ermittlung des Gleichgewichtszustandes und eine farnbedienbare Verschiebeeinrchtung (7a; 7b) zur Einstellung des Ausgleichswichts (5) vorgesehen ist, **dadurch gekennzeichnet, dass** annähernd das eine Teilgewicht (5a) dem absoluten Gewicht der Last (3) und das andere Teilgewicht (5b) dem absoluten Gewicht des Lastarmes (2) und des Ausgleichsarmes (4) zusammen mit Last (3) und dem ersten Teilgewicht (5a) entspricht, die Messeinrichtung (6) mit einem Sensor (19) zur lastabhangigen und lageunabhängigen Messung des Gleichgewichtszustandes des Stativs bei aktivierter Bremse (10) ausgebildet ist und die Messeinrichtung (6) erste Parameter an die fernbedienbare Verschiebeeinrichtung (7a) zur Einstellung des ersten Teilgewichtes (5a) übermittelt und mit einem Rechner (14), der aus diesen ersten Verschiebeparametern zweite Parameter berechnet und an die fernbedienbare Verschiebeeinrichtung (7b) zur Einstellung des zweiten Teilgewichtes (5b) übermittelt und so beide Teilgewichte (5a, 5b) automatisch justierbar sind.

2. Stativ nach Anspruch 1, **dadurch gekennzeichnet, dass** die Verschiebeeinrichtungen (7a,b) vorzugsweise über eine Kopplung (14) gekoppelt sind, wobei die Kopplung elektrisch bzw. elektronisch erfolgt und gegebenenfalls eine Software aufweist, die einen Multiplikationsfaktor, eine Formel zum Berechnen der Verschiebeparameter der gekoppelten Ausgleichsgewichte (5a, 5b) umfasst.

3. Stativ nach Anspruch 1 oder 2, **dadurch gekennzeichnet, dass** der Sensor (19) als mikromechanischer bzw. mikroelektronischer Druck-, Biege-, oder Drehmomentsensor ausgebildet ist und im Bereich der Lastaufhängung (8), des Ständers (1), am Last-, Ausgleichsarm (2,4) oder bei einer arretierbaren Schwenkachse (9) für wenigstens einen der Arme (2,4) oder bei mit erweiterten Bauteilen verbundenen Armen bzw. Schwenkachsen angeordnet sind.

4. Stativ nach einem der vorhergehenden Ansprüche, **dadurch gekennzeichnet, dass** die Verschiebeeinrichtung (7) bzw. ein Antrieb (11) dafür in Abhängigkeit von den Messwerten der Messeinrichtung (6) mit unterschiedlichen Vorschubgeschwindigkeiten betreibbar ist und dass er vorzugsweise über eine - gegebenenfalls selbsthemmende - Antriebsspindel (12) zumindest mittelbar mit dem Ausgleichsgewicht (5a) verbunden ist

5. Stativ nach einem der vorhergehenden Ansprüche, **dadurch gekennzeichnet dass** wenigstens einem Ausgleichsgewicht (5) eine Absolutpositionsbestimmungseinrichtung (70) zugeordnet ist, für das Feststellen einer Istposition des Ausgleichsgewichtes (5) relativ zum zugehörigen Ausgleichsarm, wobei diese vorzugsweise mit einem Computer (14) verbunden ist, der die Ausgangsposition des Ausgleichsgewichtes (5) bestimmt und den Antrieb (11) ansteuert zur Positionierung das Ausgleichsgewichtes (5).

6. Stativ nach einem der vorhergehenden Ansprüche, **dadurch gekennzeichnet, dass** jedem Ausgleichsgewicht (5) eine Absolutpositionsbestimmungseinrichtung (70) zugeordnet ist, und **dass** eine Steuereinrichtung vorgesehen ist, die den Antrieb (11) wenigstens eines Ausgleichsgewichtes (5) in Abhängigkeit von der Position des anderen Ausgleichsgewichtes - gegebenenfalls über Tabellen oder errechnete Faktoren - so ansteuert, dass dieses auf eine bestimmte, durch die Absolutpositionsbestimmungseinrichtung (70) erfassbare Position verschoben wird, wobei die Absolutpositionsbestimmungseinrichtung vorzugsweise einen lichtoptischen Sensor (71) und einen Markierungscode (7) umfasst.

7. Stativ mit wenigstens einer Parallelführung (15) für einen Lastarm (2a) und wenigstens einer zweiten, vertikalen Parallelführung (16) für einen Ausgleichsarm (4a), wobei beide Führungen (15,16) miteinander gekoppelt sind, nach einem der vorhergehenden Ansprüche, **dadurch gekennzeichnet, dass** die Messeinrichtung (6) im Bereich der vertikalen Parallelführung (15) angeordnet ist und über eine Steuerung (14) je einen - vorzugsweise elekstrichen - Antrieb (11a,b) zur Verschiebung von wenigstens je einem Ausgleichsgewicht (5b,c) auf der ersten und der zweiten Parallelführung (15,16) oder auf zwei unterschiedlichen Armen einer der beiden Parallelführungen (15,16) aufweist.

8. Stativ mit wenigstens einer Parallelführung (15) für den Lastarm (2a) und wenigstens einer zweiten Parallelführung (16) für den Ausgleichsarm (4a) und wenigstens einer dritten Parallelführung (17) für eine Bewegungsübertragung zwischen erster und zweiter Parallelführung (15,16), wobei wenigstens ein Verstellantrieb (11) für die Verstellung der Geometrie der zweiten Parallelführung (16) vorgesehen ist, nach einem der vorhergehenden Ansprüche, **dadurch gekennzeichnet, dass** dem Verstellantrieb (11) eine Messeinrichtung (6) - bevorzugt in einer Vertikalschwenkachse (18) der dritten Parallelführung (17) zugeordnet ist, die in Abhängigkeit von Gewichtsänderungen an der Last (3) die Geometrie der dritten Parallelführung (17) zur Schwerpunktsverlagerung des Ausgleichsgewichtes (5) in bezug auf die Vertikalschwenkachse (18) verändert.

9. Stativ mit zwei verstellbaren Ausgleichsgewichten (5) nach einem der vorhergehenden Ansprüche, **dadurch gekennzeichnet, dass** eine Kopplung der beiden Ausgleichsgewichte (5) über eine formelgestützte elektronische Steuerung (14) und je einen Antrieb (11) erfolgt wobei vorzugsweise zusätzlich eine Einstellvorrichtung (22) für das manuelle Vornehmen einer Grundeinstellung in Abhängigkeit vom Gewicht der Last (3) von Hand oder mittels des Antriebes (11) vorgesehen ist, und dass eine Felneinstellung mittels elektronischer messeinrichtung (6), Steuerung (14) und elektrischem Antrieb (11) durchführbar ist.

10. Stativ nach einem der vorhergehenden Ansprüche, **dadurch gekennzeichnet dass** anstelle oder zusätzlich zur Messeinrichtung (6) direkt oder indirekt mit einem der Arme (2,4) ein mechanischer Messwertgeber verbunden ist, insbesondere in Form eines Weggebers vorgesehen ist, der gleichzeitig als Anschlag zum Schutz vor zu grosser mechanischer Verformung der Messeinrichtung mit einem Gegenanschlag zusammenwirkt, und/oder gegebenenfalls zusätzlich als Weggeber für einen elektrischen Endschalter, der mit dem Verstellantrieb verbunden ist, ausgebildet ist, wobei vorzugsweise zwischen Weggeber und Schalter eine, gegebenenfalls anstellbare, Feder angeordnet ist, und/oder dass vorzugsweise der Schalter als Steuerglied einen elektrischen Antrieb zur - bevorzugten Not- bzw. Sicherheitsverstellung wenigstens eines Ausgleichsgewichtes (5) ansteuert

11. Stativ nach einem der vorhergehenden Ansprüche, **dadurch gekennzelohnet, dass** zusätzlich zu allfälligen messwertunterstützten Verstelleinrichtungen für Ausgleichsgewichte eine handbetätigbare, gegebenenfalls fernwirkende Antriebssteuerung und/oder eine stromlose Notlaufhandeinstellvorrichtung vorgesehen ist.

12. Stativ nach einem der vorhergehenden Ansprüche, **dadurch gekennzeichnet, dass** die Steuerung (14) des Verschiebsantriebs (11) mit der Bremseinrichtung (10) gekoppelt und gegebenenfalls mit einer Zeitverzögerungsschaltung versehen ist, so dass eine Messung und/oder Verschiebung nur bei angezogener Bremseinrichtung (10) - gegebenenfalls zeitverzögert - erfolgt.

13. Stativ nach einem der vorhergehenden Ansprüche, **dadurch gekennzeichnet dass** im Bereich der Last (3) bzw. Lastaufhängung (8) weitere automatische Verschiebeeinrichtungen für die Last (3) oder Ausgleichsgewichte (5) an der Last (3) mit einer bevorzugten Wirkrichtung senkrecht auf die Ebene, in der die Arme (2,4) liegen, angeordnet sind.

14. Stativ insbesondere für Operationsmikroskope, nach einem der vorhergehenden Ansprüche, **dadurch gekennzeichnet, dass** Teile, insbesondere Arme des Stativs aus faserverstärkten Verbundstoffen aufgebaut sind, beispielsweise aus glasfaserverstärktem Kunststoff, GFK, Karbon bzw, Kohlenstoffasern, Aramid- oder Kevlar-Fasern.

15. Stativ nach einem der vorhergehenden Ansprüche, **dadurch gekennzeichnet, dass** der Ständer (1) - gegebenenfalls teleskopartig - relativ zum Fuss (23) höhenverstellbar und/oder kippbar ist und/oder dass wenigstens eines von mehreren Radem (25) des Fusses (23) gegenüber diesem - insbesondere fernbedienbar - über das Mass üblicher Nivellierung einziehbar ist, wobei vorzugsweise bei einem Ständer (1), der gegenüber dem Fuss (23) drehbar gelagert ist, dieser via ein Kreuzrollenlager (24) gegenüber dem Fuss (23) abgestützt ist.

16. Stativ mit elektromagnetisch lösbaren Bremsen in den Gelenken, nach einem der vorhergehenden Ansprüche, **dadurch gekennzeichnet, dass** dem Elektromagneten (50) eine Schaltung zugeordnet ist, die beim Stromfreimachen des Elektromagneten (50 die Spannung an der Spule des Elektromagneten (50) stufenförmig oder nach einer Rampe abbaut, und/oder dass als Andrückelement für den eingebremsten Zustand der Bremsen (10) ein Permanentmagnet (48) vorgesehen ist, dessen Magnetismus durch den Elektromagnet (50) für den gelösten Zustand aufhebbar ist.

17. Stativ, nach einem der vorhergehenden Ansprüche, **dadurch gekennzeichnet, dass** der Lastarm (2) von der Horizontalschwenkachse (9) bis zur Last (3) gleich lang, vorzugsweise identisch geformt ausgebildet ist, wie der ihn tragende vertikale Schwenkarm (40a) von der Vertikalschwenkachse (18) bis zur Horizontalschwenkachse (9), wobei beide gegebenenfalls in zueinander parellelen Ebenen liegen und an der Horizontalschwenkachse (9) miteinander verbunden sind.

18. Verfahren zum Ausbalancieren eines Stativs, insbesondere für ein operationsmikroskop mit einem Ständer (1), mit einer Vertikalschwenkechse (18), um die das Stativ aus einer vertikalen Ebene schwenkt, mit einer Horizontalechwenkachse (9), um die das Stativ aus einer horizontalen Ebene schwenkt und mit einem die Vertikalschwenkachse (18) und die Horizontalsohwenkachse (9) verbindenden Schwenkständer (40) und mit wenigstens einem Lastarm (2) für die Last (3) und wenigstens einem Ausgleichsarm (4) für ein elektrisch fernsteuerbares Ausgleichsgewicht (5), das entlang des Ausgleichsarms (4) mittels Verschiebeeinrichtung (7) verschieblich ist, um Gewichte- oder Lageänderungen am oder des Lastarms (2) gegenüber dem Ständer (1) auszubalancieren, wobei ein zweigetelltes Ausgleichsgewicht (5a,5b) vorgesehen ist und wobei durch eine Messeinrichtung (6) das Gewicht oder die Gewichtsanderung der Last (3) gemessen wird, worauf ausgehend von dem Messwert eines der Ausgleichsgewichte (5a) an seinem Ausgleichsarm verschoben wird, um automatisch die Gewichtsänderung der Last (3) zu kompensieren, **dadurch gekennzeichnet, dass** die Messung unabhängig von der Lage der Last (3) erfolgt, wobei nach dem Ermitteln des ersten Messwertes ein zweiter Verschiebeparameter mittels Formel oder Tabelle für das zweite Ausgleichsgewicht (5b) generiert wird, worauf dieses Ausgleichsgewicht (5b) in eine Lage verschoben wird, in der es das Gewicht der Last (3) und des Lastarmes (2) mit dem Ausgleichsam oder die sich daraus ergebenden Biege- oder daraus resultierenden Drehmomente bei einer Schwenkung um die Vertikalschwenkachse (18) auszugleichen imstande ist oder die Balance des Stativs über dem Ständer (1) herzustellen imstande ist, wobei zusätzlich oder alternativ mittels einer Messeinrichtung (6b,c,e) der Druck in oder an zug- oder druckbelasteten Bauteilen des Stativs unabhängig von deren jeweiliger Lage gemessen wird, und/oder dass mittels Messeinrichtung (6a,f) ein Biegemoment an oder zwischen zwei Bauteilen des Stativs unabhängig von deren Lage gemessen wird, und/oder mittels Messeinrichtung (6d) ein Drehmoment an oder zwischen zwei Bauteilen des Stativs, insbesondere um eine während der Messung festgehaltene Schwenkachse eines Armes (2,4), unabhängig von der Winkellage dieses Armes (2,4) gemessen wird.

19. Verfahren nach Anspruch 18, **dadurch gekennzeichnet, dass** der Messwert der Messeinrichtung (6) mittels Messwertwandlers, Steuerung oder Computer (14) in wenigstens eine Steuergrösse für wenigstens einen elektrischen Antrieb (11) umgewandelt und diesem Antrieb (11) zugeführt wird, worauf der Antrieb (11) das Ausgleichsgewicht (5) - vorzugsweise in Abhängigkeit vom Messwert - in wenigstens zwei unterschiedlichen Richtungen und Geschwindigkeiten verschiebt, sowie gegebenenfalls in der neuen Position z.B. mittels Bremsen fixiert.

20. Verfahren zum Verstellen von zwei voneinander unabhängigen Ausgleichsgewichten (5a, 5b), wobei das eine Ausgleichsgewicht (5a) für den Ausgleich eines geänderten statischen Gewichts der Last (3) an ihrem horizontalen Lastarm oder Ausgleichsarm und das andere Ausgleichsgewicht (5b) für den Ausgleich des Gewichts des horizontalen Lastarms und der Last sowie des ersten Ausgleichsgewichts während des Schwenkens der Vertikalbauteile eines Stativs dient, wobei durch eine Messeinrichtung (6) die statische Gewichtsänderung der Last (3) als Messwert ermittelt wird und automatisch das eine Ausgleichsgewicht (5a) verschoben wird, **dadurch gekennzeichnet, dass** annähernd das eine Teilgewicht (5a) so gewählt wird, dass es dem absoluten Gewicht der Last (3) entspricht, und das andere Teilgewicht (5b) so gewählt wird, dass es dem absoluten Gewicht des Lastarms (2) und des Ausgleichsarms (4) zusammen mit Last (3) und dem ersten Teilgewicht (5a) entspricht, und dass dieser Messwert in eine Rechnung eingebracht wird, mit der die erforderliche Ausgleichsposition des anderen Ausgleichsgewichts (5b) berechnet wird, worauf das andere Ausgleichsgewicht (5b), mittels elektrischem Antrieb, in diese Ausgleichsposition verschoben wird.

## Claims

1. A stand for the purpose of supporting a load (3), in particular a surgical microscope, with an upright (1) with at least one load bearing arm (2) for the load (3) and at least one compensating arm (4) for a compensating weight (5) and with at least one axis of rotation (18), about which the load bearing arm (2) and the compensating arm (4) are pivotably mounted, wherein
a brake (10) is provided to lock the movement of the load bearing arm (2) and of the compensating arm about the axis of rotation (18), and the compensating weight (5) is designed such that it can be displaced along the compensating arm (4) and is subdivided into two part weights (5a, 5b) and, for the purpose of automatic balancing of weight changes on the load bearing arm (2) or of the load bearing arm (2) with respect to the upright (1), a measuring device (6) is provided to determine the state of equilibrium, and a remotely operated displacement device (7a; 7b) is provided for the purpose of adjusting the compensating weight (5),
**characterised in that** one part weight (5a) approximately corresponds to the absolute weight of the load (3), and the other part weight (5b) corresponds to the absolute weight of the load bearing arm (2) and the compensating arm (4), together with load (3) and the first part weight (5a), the measuring device (6) is constructed with a sensor (19) for the purpose of a load-dependent and position-independent measurement of the state of equilibrium of the stand with the brake (10) activated, and the measuring device (6) transmits first parameters to the remotely-operable displacement device (7a) for the purpose of adjusting the first part weight (5a), and with a computer (14), which calculates second parameters from these first displacement parameters and transmits said parameters to the remotely-operable displacement device (7b) for the purpose of adjusting the second part weight (5b), and thus both part weights (5a, 5b) can be adjusted automatically.

2. The stand according to Claim 1,
**characterised in that** the displacement devices (7a, b) are preferably coupled via a coupling means (14), wherein
the coupling is carried out electrically or electronically and possibly has software, which comprises a multiplication factor, a formula for the purpose of calculating the displacement parameters of the coupled compensating weights (5a, 5b).

3. The stand according to Claim 1 or 2,
**characterised in that** the sensor (19) is constructed as a micromechanical or microelectronic pressure, flexural or torque sensor, and is arranged in the area of the load suspension (8), of the upright (1), on the load bearing arm, compensating arm (2, 4), or on a lockable pivot axis (9) for at least one of the arms (2, 4), or on arms or pivot axes connected to expanded components.

4. The stand according to one of the preceding claims,
**characterised in that** the displacement device (7) or a drive (11) for this purpose can be operated at different advance speeds as a function of the measured values from the measuring device (6),
and **in that** the said drive (11) is connected at least indirectly to the compensating weight (5a), preferably via a drive spindle (12), if appropriate a self-locking drive spindle.

5. The stand according to one of the preceding claims,
**characterised in that** at least one compensating weight (5) is assigned an absolute position determining device (70), for determining an actual position of the compensating weight (5) relative to the associated compensating arm, wherein
the said device is preferably connected to a computer (14), which determines the initial position of the compensating weight (5) and activates the drive (11) for the purpose of positioning the compensating weight (5).

6. The stand according to one of the preceding claims,
**characterised in that** each compensating weight (5) is assigned an absolute position determining device (70),
and **in that** a control device is provided, which activates the drive (11) of at least one compensating weight (5) as a function of the position of the other compensating weight - possibly via tables or calculated factors - in such a way that the said compensating weight is displaced to a specific position, which can be registered by the absolute position determining device (70), wherein
the absolute position determining device preferably comprises an optical sensor (71) and a marking code (7).

7. A stand with at least one parallel guide (15) for a load bearing arm (2a) and at least a second, vertical parallel guide (16) for a compensating arm (4a), wherein
the two guides (15, 16) are coupled to each other, according to one of the preceding claims,
**characterised in that** the measuring device (6) is arranged in the area of the vertical parallel guide (15) and via a controller (14), in each case has a preferably electrical drive (11a, b) for the purpose of displacing in each case at least one compensating weight (5b, c) on the first and the second parallel guide (15, 16) or on two different arms of one of the two parallel guides (15, 16).

8. A stand with at least one parallel guide (15) for the load bearing arm (2a) and at least a second parallel guide (16) for the compensating arm (4a) and at least a third parallel guide (17) for a transmission of movement between the first and second parallel guides (15, 16), wherein
at least one displacement drive (11) for the displacement of the geometry of the second parallel guide (16) is provided, according to one of the preceding claims,
**characterised in that** the displacement drive (11) is assigned a measuring device (6) - preferably in a vertical pivot axis (18) of the third parallel guide (17), which, as a function of weight changes on the load (3), changes the geometry of the third parallel guide (17) in relation to the displacement of the centre of gravity of the compensating weight (5) with reference to the vertical pivot axis (18).

9. A stand with two adjustable compensating weights (5) according to one of the preceding claims,
**characterised in that** the two compensating weights (5) are coupled via a formula-assisted electronic controller (14) and a drive (11) in each case, wherein
an adjusting device (22) for the manual performance of a basic setting on the basis of the weight of the load (3) by hand or by means of the drive (11) is preferably additionally provided, and a fine adjustment can be carried out by means of the electronic measuring device (6), controller (14) and electric drive (11).

10. The stand according to one of the preceding claims,
**characterised in that** instead of or in addition to the measuring device (6), a mechanical measured value transmitter is connected, directly or indirectly, to one of the arms (2, 4), in particular is provided in the form of a distance transmitter which, at the same time, as a stop to protect against excessively large mechanical deformation of the measuring device, interacts with an opposing stop, and/or is possibly additionally constructed as a distance transmitter for an electric end switch which is connected to the displacement drive, wherein
a spring, possibly an adjustable spring, is preferably arranged between distance transmitter and switch, and/or the switch, as a control element, preferably activates an electric drive for the preferred emergency or safety displacement of at least one compensating weight (5).

11. The stand according to one of the preceding claims,
**characterised in that** in addition to any measured value-aided displacement devices for compensating weights, a hand-operated, possibly remotely acting drive controller and/or a power-free emergency manual adjusting device is provided.

12. The stand according to one of the preceding claims,
**characterised in that** the controller (14) of the displacement drive (11) is coupled to the braking device (10) and possibly provided with a time delay circuit, so that a measurement and/or displacement is carried out - possibly with a time delay - only when the braking device (10) is activated.

13. The stand according to one of the preceding claims,
**characterised in that** in the area of the load (3) or load suspension (8) there are arranged further automatic displacement devices for the load (3) or compensating weights (5) on the load (3), with a preferred effective direction at right angles to the plane in which the arms (2, 4) lie.

14. The stand, in particular for surgical microscopes, according to one of the preceding claims,
**characterised in that** parts, in particular arms, of the stand are built up from fibre-reinforced composite materials, for example from glass-fibre reinforced plastic, GRP, carbon fibres, aramid or Kevlar fibres.

15. The stand according to one of the preceding claims,
**characterised in that** the upright (1) can be adjusted vertically - possibly in the manner of a telescope - relative to the foot (23) and/or can be tilted,
and/or **in that** at least one of a plurality of wheels (25) of the foot (23) can be retracted with respect to the latter - in particular under remote control - beyond the extent of conventional levelling, wherein
in the case of an upright (1), which is rotatably mounted with respect to the foot (23), the said stand is preferably supported with respect to the foot (23) via a crossed-roller bearing (24).

16. A stand having electromagnetically releasable brakes in the joints, according to one of the preceding claims,
**characterised in that** the electromagnet (50) is assigned a circuit which, when the electromagnet (50) is deenergised, dissipates the voltage on the coil of the electromagnet (50) in steps or in accordance with a ramp,
and/or **in that** a permanent magnet (49) is provided as a pressure element for the braked state of the brakes (10), whose magnetism can be cancelled by the electromagnet (50) for the released state.

17. The stand according to one of the preceding claims,
**characterised in that** the load bearing arm (2) from the horizontal pivot axis (9) as far as the load (3) is constructed to be as long as, preferably shaped identically to, the vertical pivot arm (40a) carrying it from the vertical pivot axis (18) as far as the horizontal pivot axis (9), wherein
the two possibly lie in mutually parallel planes and are connected to each other at the horizontal pivot axis (9).

18. A method of balancing a stand, in particular for a surgical microscope with an upright (1), with a vertical pivot axis (18) about which the stand pivots from a vertical plane, with a horizontal pivot axis (9) about which the stand pivots from a horizontal plane, and with a pivoting upright (40), which connects the vertical pivot axis (18) and the horizontal pivot axis (9), and with at least one load bearing arm (2) for the load (3) and at least one compensating arm (4) for an electrically remotely controlled compensating weight (5), which can be displaced along the compensating arm (4) by means of a displacement device (7) in order to balance out weights or position changes on or of the load bearing arm (2) with respect to the upright (1), wherein
a compensating weight (5a, 5b) divided into two parts is provided, and wherein
the weight or the weight change of the load (3) is measured by means of a measuring device (6), whereupon, on the basis of the measured value, one of the compensating weights (5a) is displaced on its compensating arm in order to compensate automatically for the weight change of the load (3),
**characterised in that** the measurement is carried out independently of the position of the load (3), wherein
a second displacement parameter is generated for the second compensating weight (5b) by means of a formula or table following the determination of the first measured value, whereupon this compensating weight (5b) is displaced into a position in which it is able to compensate for the weight of the load (3) and of the load bearing arm (2) with the compensating arm or the flexural moments which result from this or the torques which result from this during pivoting about the vertical pivot axis (18), or is able to produce the balance of the stand via the upright (1), wherein
in addition or alternatively, by means of a measuring device (6b, c, e), the pressure in or on tension-loaded or compression-loaded components of the stand is measured independently of their respective position,
and/or **in that** by means of a measuring device (6a, f), a flexural moment on or between two components of the stand is measured independently of their position, and/or by means of a measuring device (6d), a torque on or between two components of the stand, in particular about a pivot axis of an arm (2, 4) which is held firmly during the measurement, is measured independently of the angular position of this arm (2, 4).

19. The method in accordance with Claim 18,
**characterised in that** the measured value from the measuring device (6) is converted by means of a measured-value converter, controller or computer (14) into at least one control variable for at least one electric drive (11) and is supplied to this drive (11), whereupon the drive (11) displaces the compensating weight (5) - preferably as a function of the measured value - in at least two different directions and at at least two different speeds, and possibly fixes it in the new position, for example by means of brakes.

20. A method of displacing two mutually independent compensating weights (5a, 5b), wherein
one compensating weight (5a) serves to compensate for a changed static weight of the load (3) on its horizontal load bearing arm or compensating arm, and the other compensating weight (5b) serves to compensate for the weight of the horizontal load bearing arm and the load and the first compensating weight during the pivoting of the vertical components of a stand, wherein
the static weight change of the load (3) is determined by means of a measuring device (6) and one compensating weight (5a) is displaced automatically,
**characterised in that** one part weight (5a) is selected in such a way that it approximately corresponds to the absolute weight of the load (3), and the other part weight (5b) is selected in such a way that it corresponds to the absolute weight of the load bearing arm (2) and of the compensating arm (4) together with load (3) and the first part weight (5a),
and **in that** this measured value is introduced into a calculation with which the requisite compensatory position of the other compensating weight (5b) is calculated, whereupon the other compensating weight (5b) is displaced into this compensating position by means of an electric drive.

## Revendications

1. Pied pour porter une charge (3), en particulier un microscope d'opération, comprenant un pied (1) avec au moins un bras de charge (2) pour la charge (3) et au moins un bras d'équilibrage (4) pour un poids d'équilibrage (5) et au moins un axe de rotation (18), autour duquel le bras de charge (2) et le bras d'équilibrage (4) sont montés pivotants, un frein (10) destiné à arrêter le mouvement du bras de charge (2) et du bras d'équilibrage autour de l'axe de rotation (18) étant prévu et le poids d'équilibrage (5) étant réalisé de manière déplaçable le long du bras d'équilibrage (4) et étant subdivisé en deux poids partiels (5a, 5b) et un dispositif de mesure (6) pour déterminer l'état d'équilibre et un dispositif de déplacement télécommandable (7a ; 7b) pour ajuster le poids d'équilibrage (5) étant prévus pour le contre-balancement automatique de variations de poids sur le bras de charge (2) ou du bras de charge (2) par rapport au support (1), **caractérisé en ce qu'**approximativement l'un des poids partiels (5a) correspond au poids absolu de la charge (3) et l'autre poids partiel (5b) correspond au poids absolu du bras de charge (2) et du bras d'équilibrage (4) conjointement avec la charge (3) et le premier poids partiel (5a), le dispositif de mesure (6) étant réalisé avec un capteur (19) pour mesurer, en fonction de la charge et indépendamment de la position, l'état d'équilibre du pied lorsque le frein (10) est activé et le dispositif de mesure (6) transmettant des premiers paramètres au dispositif de déplacement télécommandable (7a) pour l'ajustement du premier poids partiel (5a) et comprenant un ordinateur (14), qui calcule des deuxièmes paramètres à partir de ces premiers paramètres de déplacement et les transmet au dispositif de déplacement télécommandable (7b) pour l'ajustement du deuxième poids partiel (5b) et ainsi les deux poids partiels (5a, 5b) peuvent être ajustés automatiquement.

2. Pied selon la revendication 1, **caractérisé en ce que** les dispositifs de déplacement (7a, b) sont couplés de préférence par le biais d'un accouplement (14), l'accouplement s'effectuant de manière électrique ou électronique et présentant éventuellement un logiciel, qui comprend un facteur de multiplication, une formule pour calculer les paramètres de déplacement des poids d'équilibre couplés (5a, 5b).

3. Pied selon la revendication 1 ou 2, **caractérisé en ce que** le capteur (19) est réalisé sous forme de capteur micromécanique ou microélectronique de pression, de flexion ou de couple et est disposé dans la zone de la suspension de charge (8), du support (1), sur le bras de charge, le bras d'équilibrage (2, 4) ou, dans le cas d'un axe de pivotement (9) pouvant être bloqué, pour au moins l'un des bras (2, 4) ou dans le cas de bras ou d'axes de pivotement reliés à des composants élargis.

4. Pied selon l'une des revendications précédentes, **caractérisé en ce que** le dispositif de déplacement (7), respectivement un entraînement (11) pour celui-ci, peut être actionné en fonction des valeurs de mesure du dispositif de mesure (6) avec différentes vitesses d'avance et **en ce qu'**il est connecté de préférence par le biais d'une broche d'entraînement (12) - éventuellement autobloquante - au moins indirectement au poids d'équilibrage (5a).

5. Pied selon l'une des revendications précédentes, **caractérisé en ce qu'**un dispositif de détermination de position absolue (70) est associé à au moins un poids d'équilibrage (5), pour la détermination d'une position réelle du poids d'équilibrage (5) par rapport au bras d'équilibrage associé, celle-ci étant de préférence reliée à un ordinateur (14), qui détermine la position de départ du poids d'équilibrage (5) et commande l'entraînement (11) pour le positionnement du poids d'équilibrage (5).

6. Pied selon l'une des revendications précédentes, **caractérisé en ce qu'**un dispositif de détermination de position absolue (70) est associé à chaque poids d'équilibrage (5), et **en ce qu'**un dispositif de commande est prévu, lequel commande l'entraînement (11) d'au moins un poids d'équilibrage (5) en fonction de la position de l'autre poids d'équilibrage - éventuellement par le biais de tableaux ou de facteurs calculés -, de telle sorte que celui-ci soit déplacé à une position déterminée, pouvant être détectée par le dispositif de détermination de position absolue (70), le dispositif de détermination de position absolue comprenant de préférence un capteur optique (71) et un code de marquage (7).

7. Pied comprenant au moins un guide parallèle (15) pour un bras de charge (2a) et au moins un deuxième guide parallèle vertical (16) pour un bras d'équilibrage (4a), les deux guides (15, 16) étant couplés l'un à l'autre, selon l'une des revendications précédentes, **caractérisé en ce que** le dispositif de mesure (6) est disposé dans la zone du guide parallèle vertical (15) et présente par le biais d'une commande (14) à chaque fois un entraînement (11a, b) - de préférence électrique - pour le déplacement d'au moins un poids d'équilibrage (5b, c) sur le premier et le deuxième guide parallèle (15, 16) ou sur deux bras différents de l'un des deux guides parallèles (15, 16).

8. Pied comprenant au moins un guide parallèle (15) pour le bras de charge (2a) et au moins un deuxième guide parallèle (16) pour le bras d'équilibrage (4a) et au moins un troisième guide parallèle (17) pour un transfert de mouvement entre le premier et le deuxième guide parallèle (15, 16), au moins un entraînement de réglage (11) étant prévu pour le réglage de la géométrie du deuxième guide parallèle (16), selon l'une des revendications précédentes, **caractérisé en ce qu'**un dispositif de mesure (6) est associé à l'entraînement de réglage (11) - de préférence dans un axe de pivotement vertical (18) du troisième guide parallèle (17), lequel modifie, en fonction de variations du poids sur la charge (3), la géométrie du troisième guide parallèle (17) pour le déplacement de centre de gravité du poids d'équilibrage (5) par rapport à l'axe de pivotement vertical (18).

9. Pied comprenant deux poids d'équilibrage (5) réglables selon l'une des revendications précédentes, **caractérisé en ce qu'**un accouplement des deux poids d'équilibrage (5) s'effectue par le biais d'une commande (14) électronique assistée par des formules et d'un entraînement respectif (11), de préférence un dispositif de réglage (22) étant prévu en supplément pour effectuer manuellement un ajustement de base en fonction du poids de la charge (3) à la main ou au moyen de l'entraînement (11), et **en ce qu'**un ajustement fin peut être effectué au moyen du dispositif de mesure électronique (6), de la commande (14) et de l'entraînement électrique (11).

10. Pied selon l'une des revendications précédentes, **caractérisé en ce que**, à la place ou en supplément du dispositif de mesure (6), un capteur mécanique de valeur de mesure est relié directement ou indirectement à l'un des bras (2, 4), est prévu en particulier sous la forme d'un capteur de course, qui coopère simultanément, en tant que butée pour protéger contre une déformation mécanique trop importante du dispositif de mesure, avec une contre-butée, et/ou qui est réalisé éventuellement en supplément comme capteur de course pour un fin de course électrique, qui est relié à la commande de réglage, un ressort, éventuellement ajustable, étant disposé de préférence entre le capteur de course et le commutateur, et/ou **en ce que** de préférence le commutateur active en tant qu'organe de commande un entraînement électrique pour le réglage d'urgence ou de sécurité préféré d'au moins un poids d'équilibrage (5).

11. Pied selon l'une des revendications précédentes, **caractérisé en ce qu'**une commande d'entraînement actionnable manuellement, éventuellement télécommandée, et/ou un dispositif de réglage manuel d'urgence sans courant est/sont prévu(s) en plus des dispositifs de réglage éventuels assistés par des valeurs de mesure pour des poids d'équilibrage.

12. Pied selon l'une des revendications précédentes, **caractérisé en ce que** la commande (14) de l'entraînement de déplacement (11) est couplée avec le dispositif de freinage (10) et est éventuellement dotée d'un circuit de temporisation, de sorte qu'une mesure et/ou un déplacement ne s'effectue que lorsque le dispositif de freinage (10) est serré, éventuellement temporisé.

13. Pied selon l'une des revendications précédentes, **caractérisé en ce que** dans la zone de la charge (3) ou de la suspension de charge (8) sont disposés d'autres dispositifs de déplacement automatiques pour la charge (3) ou des poids d'équilibrage (5) sur la charge (3) avec une direction d'action préférée perpendiculairement au plan dans lequel se trouvent les bras (2, 4).

14. Pied en particulier pour des microscopes d'opération, selon l'une des revendications précédentes, **caractérisé en ce que** des parties, en particulier des bras du pied, sont fabriquées à base de matériaux composites renforcés de fibre, par exemple en plastique renforcé de fibre de verre, en carbone ou en fibres de carbone, en fibres d'aramide ou de kevlar.

15. Pied selon l'une des revendications précédentes, **caractérisé en ce que** le support (1) peut être réglé en hauteur et/ou basculé - éventuellement de manière télescopique -, par rapport à la base (23), et/ou **en ce qu'**au moins l'une de plusieurs roues (25) de la base (23) peut être retirée par rapport à cette base - en particulier par télécommande - au-delà de la mesure d'une mise à niveau usuelle, où, de préférence dans le cas d'un support (1), qui est monté rotatif par rapport à la base (23), celui-ci est supporté par un palier à rouleaux croisés (24) par rapport à la base (23).

16. Pied équipé de freins desserrables de façon électromagnétique dans les articulations, selon l'une des revendications précédentes, **caractérisé en ce qu'**un circuit est associé à l'électroaimant (50), lequel circuit diminue la tension au niveau de la bobine de l'électroaimant (50) par degrés ou selon une rampe lorsque l'électroaimant (50) n'est plus parcouru par un courant, et/ou **en ce qu'**on prévoit comme élément de serrage pour l'état de freinage des freins (10) un aimant permanent (49), dont le magnétisme peut être interrompu par l'électroaimant (50) pour l'état desserré.

17. Pied selon l'une des revendications précédentes, **caractérisé en ce que** le bras de charge (2) est réalisé, depuis l'axe de pivotement horizontal (9) jusqu'à la charge (3) avec la même longueur, de préférence avec la même forme que le bras de pivotement vertical (40a) qui le porte, depuis l'axe de pivotement vertical (18) jusqu'à l'axe de pivotement horizontal (9), les deux se trouvant éventuellement dans des plans parallèles entre eux et étant connectés l'un à l'autre au niveau de l'axe de pivotement horizontal (9).

18. Procédé pour l'équilibrage d'un support, en particulier pour un microscope d'opération comprenant un support (1), avec un axe de pivotement vertical (18), autour duquel le pied pivote à partir d'un plan vertical, avec un axe de pivotement horizontal (9), autour duquel le support pivote depuis un plan horizontal et avec un support de pivotement (40) reliant l'axe de pivotement vertical (18) et l'axe de pivotement horizontal (9) et avec au moins un bras de charge (2) pour la charge (3) et au moins un bras d'équilibrage (4) pour un poids d'équilibrage (5) télécommandable électriquement, qui peut être déplacé le long du bras d'équilibrage (4) au moyen du dispositif de déplacement (7), pour contrebalancer des variations de poids ou de position sur le ou du bras de charge (2) par rapport au support (1), un poids d'équilibrage en deux parties (5a, 5b) étant prévu et le poids ou la variation de poids de la charge (3) étant mesuré(e) par un dispositif de mesure (6), l'un des poids d'équilibrage (5a) étant ensuite déplacé à partir de la valeur de mesure, afin de compenser automatiquement la variation de poids de la charge (3), **caractérisé en ce que** la mesure s'effectue indépendamment de la position de la charge (3), où, après la détermination de la première valeur de mesure, un deuxième paramètre de déplacement est généré au moyen de formule ou de table pour le deuxième poids d'équilibrage (5b), ce poids d'équilibrage (5b) étant déplacé ensuite dans une position dans laquelle il est en mesure d'équilibrer le poids de la charge (3) et du bras de charge (2) avec le bras d'équilibrage ou bien les couples de flexion se produisant ou les couples de rotation en résultant lors d'un pivotement autour de l'axe de pivotement vertical (18), ou est en mesure d'effectuer l'équilibrage du pied sur le support (1), la pression dans ou sur des composants sollicités en traction ou en pression du pied étant mesurée indépendamment de leur position respective en supplément ou comme alternative au moyen d'un dispositif de mesure (6b, c, e), et/ou **en ce qu'**un couple de flexion est mesuré au moyen du dispositif de mesure (6a, f) sur ou entre deux composants du pied indépendamment de leur position, et/ou un couple de rotation est mesuré au moyen d'un dispositif de mesure (6d) sur ou entre deux composants du support, en particulier autour d'un axe de pivotement d'un bras (2, 4) fixé pendant la mesure, indépendamment de la position angulaire de ce bras (2, 4).

19. Procédé selon la revendication 18, **caractérisé en ce que** la valeur de mesure du dispositif de mesure (6) est convertie, au moyen d'un convertisseur de valeur de mesure, d'une commande ou d'un ordinateur (14), en au moins une grandeur de commande pour au moins un entraînement électrique (11) et est acheminée à cet entraînement (11), l'entraînement (11) déplaçant le poids d'équilibrage (5) - de préférence en fonction de la valeur de mesure - dans au moins deux directions différentes et suivant deux vitesses différentes, et le fixant éventuellement dans la nouvelle position, par exemple au moyen de freins.

20. Procédé destiné au réglage de deux poids d'équilibrage (5a, 5b) indépendants l'un de l'autre, l'un des poids d'équilibrage (5a) servant à l'équilibrage d'un poids statique modifié de la charge (3) sur son bras de charge horizontal ou sur son bras d'équilibrage et l'autre poids d'équilibrage (5b) servant à l'équilibrage du poids du bras de charge horizontal et de la charge ainsi que du premier poids d'équilibrage pendant le pivotement des composants verticaux d'un pied, la variation statique de poids de la charge (3) étant déterminée par un dispositif de mesure (6) comme valeur de mesure et l'un des poids d'équilibrage (5a) étant déplacé automatiquement, **caractérisé en ce que** l'un des poids partiels (5a) est choisi approximativement de telle sorte qu'il correspond au poids absolu de la charge (3), et l'autre poids partiel (5b) est choisi de telle sorte qu'il correspond au poids absolu du bras de charge (2) et du bras d'équilibre (4) conjointement avec la charge (3) et le premier poids partiel (5a), et **en ce que** cette valeur de mesure est introduite dans un calcul, avec lequel la position d'équilibre nécessaire de l'autre poids d'équilibrage (5b) est calculée, après quoi l'autre poids d'équilibrage (5b) est déplacé, au moyen d'un entraînement électrique, dans cette position d'équilibrage.
